# EUROPEAN PATENT SPECIFICATION

(11) **EP 1 992 271 B1**
(45) Date of publication and mention of the grant of the patent: **13.06.2012**
(21) Application number: 07714775.9
(22) Date of filing: 22.02.2007
(51) Int. Cl.: A61B 1/273, A61B 1/04

(54) **CAPSULE ENDOSCOPE SYSTEM**
KAPSEL-ENDOSKOPSYSTEM
SYSTEME ENDOSCOPIQUE A CAPSULE

(30) Priority: 22.02.2006 US 775536 P
(43) Date of publication of application: 19.11.2008
(73) Proprietor: Olympus Medical Systems Corp., Tokyo 151-0072 (JP)
(72) Inventor: BARLOW, David E., Coopersburg, Pennsylvania (US); KAJI, Kunihide, Shibuya-ku, Tokyo 1510072 (JP); SUZUKI, Takayuki, Shibuya-ku, Tokyo 1510072 (JP); SATO, Masatoshi, Shibuya-ku, Tokyo 1510072 (JP); SHIONO, Junji, Shibuya-ku, Tokyo 1510072 (JP); MIKKAICHI, Takayasu, Shibuya-ku, Tokyo 1510072 (JP)
(74) Representative: von Hellfeld, Axel
(86) International application number: PCT/JP2007/053277
(87) International publication number: WO 2007/097393

(56) References cited:
- WO-A1-2007/078003
- JP-A- 06 114 036
- JP-A- 55 148 541
- JP-A- 2002 360 508
- JP-A- 2003 093 332
- JP-A- 2003 135 388
- JP-A- 2004 016 504
- JP-A- 2005 103 092
- JP-A- 2005 296 063
- JP-A- 2005 534 367
- JP-U- 04 131 214
- US-A1- 2003 153 866
- US-A1- 2004 260 164
- US-A1- 2005 085 697
- US-A1- 2005 165 272

## Description

### TECHNICAL FIELD

The present invention relates to a capsule endoscope system which improves the functionality of a capsule endoscope for performing diagnostic and therapeutic activities in the human body.

### BACKGROUND ART OF THE INVENTION

Self-contained capsule devices that capture and transmit electronic images of internal lumens of the body are in common use to diagnose GI tract diseases, and in particular, diseases of the small intestine. These capsules typically contain a solid-state electronic image sensor (e.g., a CCD or CMOS imager), an objective lens for focusing images of the GI tract mucosa on the photosensitive surface of the image sensor, light-emitting diodes (LED) for illuminating the interior of the body to create the images, electronic circuitry for capturing the images and transmitting them to an external recording device, an antenna to facilitate efficient image transmission, and a battery(s) to provide power for these functions. The imaging capsule typically captures sequential still images through a transparent dome-shaped lens at one end of the capsule.
US 2004/260164 A1 discloses a system for monitoring physiological parameter in a body lumen, in particular the exophagos and more in particular the Barret's Esophagus.
US 2004/260164 A1 discloses a capsule endoscope having an internal lumen and a catheter tube connected to the capsule endoscope. In correspondence to the walls of the internal lumen, threaded means permit the connection of the distal end of the catheter tube to the capsule endoscope so that the catheter tube is communicatively connected with the internal lumen.
US 2005/085697 A1 discloses a gastrointestinal tract examining apparatus comprising a capsule endoscope removably fixed to a tether. The tether is enclosed in a tube member.
US 2003/0153866 A1 discloses a medical intraluminal device comprising a capsule having an internal lumen and a catheter tube.
US 2005/0165272 A1 discloses an endoscope system comprising a first tube endoscope transporting a separable capsule endoscope.
Imaging capsules currently on the market are typically slightly larger than the size of a large vitamin pill. The electronics of the capsule are activated, and the capsule is swallowed by the patient with a small quantity of water. Once in the stomach, the capsule typically stays there for a certain period of time until stomach contractions move it into the small intestine. Once in the small intestine, it typically moves through the small intestine and then into and through the large intestine by normal peristaltic contractions of smooth muscles within the wall of the intestines. Within 12-48 hours the capsule is typically expelled from the body during a normal bowel movement.
While in the body, the capsule captures sequential still images of the interior wall of the GI tract, typically at a rate of two images per second, as it is propelled through the digestive tract. These images are transmitted in a continuous stream by the capsule via radio frequency (RF) signals to several RF antennas placed on the patient's skin. The external antennas bring the signal to a small recording device worn around the patient's waist, where they are temporarily stored.
After the capsule has completed its transit of the area of interest (e.g., the small intestine), the recorder and antennas are removed from the patient and the recording device is connected to a computer workstation. The images stored in the recorder are electronically transferred to the computer and stored on the computer's hard disk drive. Proprietary software is then used to display these images in a manner that is convenient for a physician to review them, analyze them, and make a diagnosis regarding the portions of the GI tract traversed by the capsule. Capsule endoscopes of this design are typically used to diagnose obscure GI bleeding (OGIB) within the small bowel, Crohn' s disease, celiac sprue, and other common maladies of the GI tract.

There have been recent attempts to expand the usefulness of the wireless imaging capsule in other parts of the GI tract. Of particular interest is to use the capsule to examine the esophagus, particularly the lower portion of the esophagus (the distal esophagus) . A common desire is to use the wireless capsule to diagnose a condition of the distal esophagus known as Barrett's esophagus. Barrett's esophagus is of particular medical concern because it is linked to an increased risk of esophageal cancer.

Several clinical studies have been published on the use of a commercial wireless capsule designed for diagnosis within the small intestine, as a tool for examining the distal esophagus. These clinical studies have found that when the capsule is swallowed it passes very quickly through the esophagus into the stomach. So quickly in fact, that few images are captured of the distal esophagus and its junction with the stomach - the area particularly affected by Barrett's esophagus. It has been reported that the imaging time of the area of interest is only a matter of seconds, and that often the images obtained are less than ideal because saliva obscures the image. Several attempts have been made to improve capsule endoscope observation of the esophagus. One modification is to place the patient in a slightly recumbent position, rather than sitting upright, as the capsule is swallowed. The intention is to slow the speed at which the capsule passes through the esophagus into the stomach.

In addition to modifying the swallowing procedure, modifications to the device itself have been made to increase the potential number of images captured in the esophagus during passage of the capsule. One modification has been to increase the frequency at which images are captured. One commercial wireless capsule specifically designed for use in the esophagus captures images at 4 frames/second, compared to the 2 frames/second rate of the capsule designed for use in the small intestine. Another modification has been to add a second imaging system to the capsule. Small bowel capsules typically image from only one end of the capsule, whereas specially designed esophageal capsules may have two imaging systems, one observing from one end of the capsule, the other observing from the opposite end of the capsule.

Even with these modifications, the capsule makes a single pass through the esophagus, and if the area (s) of interest are not adequately seen, there is no recourse to go back and observe the region of interest again. Because of this limitation, several researchers have tried attaching a thin string to the capsule to control its descent through the esophagus - indeed to even stop or reverse its direction by pulling on the proximal end of the string which extends from the patient' s mouth. A recent report of such experiments was published by Ramirez et al. (refer to non patent document 1 as mentioned below). Ramirez reports success tying 4 strings around a wireless capsule, having the patient swallow the capsule, and then pulling the capsule from the stomach back into the esophagus for a second and third (partial) re-swallow. At the end of the examination the capsule is removed from the patient by slowly pulling on the ends of the strings extending from the patient's mouth. Not only was the wireless capsule capable of being retrieved from the patient for reuse on a subsequent patient, but controlling the position of the capsule via the strings tied to it allowed for extended and more complete visualization of the esophagus - in particular the distal esophagus, thus aiding in the patient's diagnosis. The string capsule procedure was performed with the patient in a sitting position, without sedation or topical anesthetic.
NON PATENT DOCUMENT 1: "Feasibility and safety of string, wireless capsule endoscopy in the diagnosis of Barrett's esophagus." Ramirez FC, Shaukat MS, Young MA, Johnson DA, Akins R. Gastrointestinal Endoscopy, vol. 61(6), pgs 741-746, 2005.

### DETAILED DESCRIPTION OF THE INVENTION

### PROBLEMS TO BE SOLVED BY THE INVENTION

Despite the improved performance of the string capsule over a free-floating capsule, the authors report several problems with the device. Some patients had difficulty with the initial swallow of the capsule due to its large size. Visualization of esophageal tissue was often obstructed by saliva and bubbles. Some patients reported throat discomfort and gagging due to the presence of the strings. Capsule retrieval was difficult and uncomfortable in some patients due to difficulty withdrawing the capsule through the upper esophageal sphincter (UES) due to spasms of the UES.

An object of the present invention is to provide a system which not only improves the functionality of a swallowing-type capsule endoscope but also supplies fluids such as water and air via a capsule endoscope to and removes them from the body of a patient in order to improve the quality of an examination.

Another object of the present invention is to provide a system which is capable of supplying fluids such as water, air and gas into the body of a patient via a swallowing-type capsule endoscope and also connecting a catheter tube to the capsule endoscope in order to suction these fluids via the capsule endoscope.

A further object of the present invention is to provide a system for washing the lens of an imaging system of a swallowing-type capsule endoscope.

Yet another object of the present invention is to provide a system for relieving a patient of discomfort during an examination.

### MEANS FOR SOLVING THE PROBLEM

A capsule endoscope system of the present invention includes: a capsule endoscope having an internal lumen; a catheter tube connected to the capsule endoscope; and a connection portion for connecting the distal end of the catheter tube to the capsule endoscope so that the catheter tube is communicatively connected with the internal lumen of the capsule endoscope.

The capsule endoscope system of the present invention may additionally includes a string tether of which one end thereof is connected to the capsule endoscope. The catheter tube is advanced along the string tether, with the string tether being passed from the one end into the intraperitoneal cavity, and thereby guided up to the capsule endoscope.

In the capsule endoscope system of the present invention, a capsule endoscope is located inside the body of a patient, a catheter tube is then inserted into the body, with the proximal end thereof being left outside the body, and the distal end of the catheter tube being connected to the capsule endoscope in such a way that the catheter tube is communicatively connected with the internal lumen of the capsule endoscope.

According to the capsule endoscope system of the present invention, it is possible to supply fluids such as water, air and gas to an organ of the body in which the capsule endoscope is located via a catheter tube and a capsule endoscope. Thereby, an appropriate treatment can be provided at various aspects of procedures. For example, in a case where the capsule endoscope is disposed into the esophagus, air is injected into the esophagus via the catheter tube and the capsule endoscope to dilate the esophagus. Thereby, it is possible to secure a larger visual range inside the esophagus. Furthermore, air is injected into the esophagus to expand the esophagus wall, by which the lower esophageal sphincter is distended to facilitate a favorable view of the portion concerned.

Water is injected via a catheter tube and a capsule endoscope and discharged from the leading end of the capsule endoscope. Thereby, it is possible to wash away body tissues attached on an imaging system mounted at the leading end of the capsule endoscope in general or saliva and bubbles attached on the capsule endoscope to obscure an image.

Liquids and fluids such as air and gas are suctioned and removed via a catheter tube and a capsule endoscope from the capsule endoscope. Thereby, it is possible to discharge water injected for removing body tissue and saliva. Further, it is possible to remove saliva and bubbles attached on the capsule endoscope which obscures images from the inside of the body.

In the capsule endoscope system of the present invention, a distal opening of an internal lumen on the capsule endoscope may be directed so that fluid supplied via the internal lumen can be discharged in the direction along the leading end face of the capsule endoscope. Fluids such as water, air and gas are discharged from the distal opening of the internal lumen, thereby it becomes possible to wash away saliva and bubbles attached on the capsule endoscope more effectively.

In the capsule endoscope system of the present invention, the connection portion may include: a dilation member mounted at the distal end of the catheter tube and dilating as appropriate; and an acceptance portion mounted on the internal lumen of the capsule endoscope and in which the acceptance portion accepts the dilation portion. The dilation portion is dilated within the acceptance portion and thereby the dilation portion is engaged with the acceptance portion. Thereby, the distal end of the catheter tube is connected to the capsule endoscope so as not to be easily disengaged therefrom.

The dilation member may be a balloon which inflates by injection of a fluid or a high-molecular absorbent which dilates by supply of water.

Furthermore, the dilation portion may be a coil mounted at the distal end of the catheter tube and which expands the outer diameter thereof when being driven in a compressing or twisting manner. It is preferable that the capsule endoscope system of the present invention include a coil driving member for rotating the coil in a compressing or twisting manner. The coil as a dilation member is rotated in a compressing or twisting manner via the coil driving member and engaged with an acceptance portion by expansion of the outer diameter inside the acceptance portion.

The coil driving member may be an over tube disposed outside the catheter tube so that the catheter tube is passed through the overtube. It is preferable that one end of the coil be fixed to the distal end of the catheter tube, while the other end of the coil is fixed to the distal end of the overtube. A relative movement or rotation of the overtube with respect to the catheter tube allows the coil to expand in the outer diameter, thereby resulting in an easy deformation of the coil.

In the capsule endoscope system of the present invention, the connection portion may include: an elastically deformable snap portion mounted at one of the distal end of the catheter tube and the capsule endoscope; and an acceptance portion mounted at the other of the distal end of the catheter tube or the capsule endoscope and hooked upon acceptance of the snap portion. When accepted at the acceptance portion, the snap portion is hooked onto the acceptance portion by its own elastic force. Thereby, the distal end of the catheter tube is connected to the capsule endoscope so as not to be easily disengaged therefrom.

In the capsule endoscope system of the present invention, the connection portion may include: a magnet mounted at one of the distal end of the catheter tube and the capsule endoscope, and a magnetic body mounted at the other of the distal end of the catheter tube and the capsule endoscope and attracted by the magnet. The magnet attracts the magnetic body, by which the distal end of the catheter tube is connected to the capsule endoscope so as not to be easily disengaged therefrom.

The magnet may be an electro-magnet or a permanent magnet.

Furthermore, the magnetic body may be a permanent magnet. It is preferable that the permanent magnet mounted at the distal end of the catheter tube is different in polarity depending on two regions divided by the central axis of the catheter tube and the permanent magnet mounted at the capsule endoscope is different in polarity depending on two regions divided by the center of the face firmly attached to the permanent magnet mounted at the distal end of the catheter tube. A permanent magnet attracts another permanent magnet, by which the distal end of the catheter tube is connected to the capsule endoscope so as not to be easily disengaged therefrom. Furthermore, where the catheter tube is rotated with respect to the capsule endoscope, these two permanent magnets are repelled by each other, therefore, these magnets with the same polarity face each other. Thereby, the distal end of the catheter tube can be disengaged from the capsule endoscope.

In the capsule endoscope system of the present invention, a positioning portion for positioning the catheter tube with respect to the capsule endoscope may be mounted at the connection portion so that the catheter tube is communicatively connected with the internal lumen of the capsule endoscope. When the catheter tube is connected to the capsule endoscope, the positioning portion acts to communicatively connect the catheter tube with the internal lumen of the capsule endoscope, thereby it becomes possible to secure procedures such as the supply of air and water or the discharge thereof via the catheter tube and the internal lumen of the capsule endoscope.

In the capsule endoscope system of the present invention, the capsule endoscope may be tapered toward the proximal end to which the catheter tube is connected. For example, where the capsule endoscope inserted from the mouth through the esophagus is moved in a retrograde fashion so as to pull a string tether and a catheter tube out of the mouth, the tapered proximal end of the capsule endoscope allows the outer diameter of the catheter tube and that of the capsule endoscope to change smoothly not abruptly. Therefore, the capsule endoscope can be passed through anatomically narrow channels such as the lower esophageal sphincter and the upper esophageal sphincter smoothly without being caught therein. Thus, a patient is relieved of discomfort.

The capsule endoscope system of the present invention may additionally includes with an operation portion mounted at the proximal end of the catheter tube and operating at least any one of the procedures of air supply, water supply and suction via the catheter tube and a capsule endoscope connected to the catheter tube. An operator is thereby able to perform at least any one of the procedures of air supply, water supply and suction by using the operation portion.

In the capsule endoscope system of the present invention, the connection portion may removably connect the distal end of the catheter tube to the capsule endoscope. The distal end of the catheter tube is disengaged from the capsule endoscope, whenever necessary, thereby facilitated is the retrieval of the capsule endoscope from the body of a patient. Therefore, there is less physical burden on a patient resulting from the procedure.

### ADVANTAGEOUS EFFECTS OF THE INVENTION

The present invention is able to increase the functionality of a swallowing-type capsule endoscope and improve the quality of an examination.
Furthermore, the present invention is able to connect a catheter tube to a swallowing-type capsule endoscope, supply fluids such as water, air, and gas into the body of a patient via the capsule endoscope and suction the fluids.
Still furthermore, the present invention is able to wash the lens of an imaging system used in the swallowing-type capsule endoscope.
In addition, the present invention is able to relieve a patient of discomfort during an examination.

### BRIEF DESCRIPTION OF THE DRAWINGS

FIG. 1 is a schematic sectional view of the body of a patient from a frontal view, illustrating a state that a wireless imaging capsule connected to a string tether is passed through the lower esophagus.
FIG. 2 is a schematic sectional view of the body of a patient from a frontal view, illustrating a state that a catheter tube is advanced to the lower esophagus along the string tether.
FIG. 3 is a schematic sectional view of the body of a patient from a frontal view, illustrating a state that a mouth piece is attached to the oral cavity of the patient for guiding the catheter tube.
FIG. 4 is a schematic view illustrating a whole constitution of a capsule endoscope system.
FIG. 5 is a perspective view illustrating the wireless imaging capsule and the distal end of the catheter tube when observed in a forward oblique direction.
FIG. 6. is a perspective view illustrating the wireless imaging capsule and the distal end of the catheter tube when observed from a backward oblique direction.
FIG. 7 is a schematic sectional view illustrating the wireless imaging capsule and the string tether connected to the capsule.
FIG. 8 is a schematic sectional view illustrating a state that the catheter tube is advanced to the wireless imaging capsule along the string tether.
FIG. 9 is a schematic sectional view illustrating a state that the distal end of the catheter tube is connected to the wireless imaging capsule.
FIG. 10 is a schematic sectional view illustrating an exemplified variation of the wireless imaging capsule.
FIG. 11 is a perspective view illustrating a controller constituting the capsule endoscope system.
FIG. 12 is a perspective view illustrating a fixed portion of the string tether mounted on the controller.
FIG. 13 is a partial cutaway view illustrating an internal structure of the controller.
FIG. 14 is a sectional view of a valve mechanism incorporated into the controller, illustrating a state that the valve is closed.
FIG. 15 is a sectional view of a valve mechanism incorporated into the controller, illustrating a state that the valve is opened by operating a button.
FIG. 16 is a perspective view illustrating a mouthpiece attached into the oral cavity of a patient for guiding the catheter tube.
FIG. 17 is a sectional view illustrating the mouthpiece taken along line A to A of FIG. 16.
FIG. 18 is a schematic sectional view of the body of a patient from a frontal view, illustrating a state that the dilation tube is advanced along the catheter tube to the lower esophagus.
FIG. 19 is a sectional viewillustrating the stringtether, the catheter tube and the dilation tube taken along line B to B in FIG. 18.
FIG. 20 is a schematic transverse sectional view of the anterior abdomen of a patient, illustrating a capsule endoscope system which is used together with another operative instrument piercing the abdominal wall of a patient.
FIG. 21 is a schematic transverse sectional view of the anterior abdomen of a patient, illustrating a state that the wireless imaging capsule is disposed into the abdomen via a trocar piercing the abdominal wall.
FIG. 22 is a schematic transverse sectional view of the anterior abdomen of a patient, illustrating a state that the wireless imaging capsule is disposed into the abdomen via a flexible endoscope piercing the abdominal wall.
FIG. 23 is a view illustrating an exemplified variation of the capsule endoscope: system or a partial cutaway view of a system in which a connection portion of connecting the catheter tube to the wireless imaging capsule is composed of a balloon and an acceptance portion.
FIG. 24 is a partial cutaway view illustrating a state that the balloon is engaged with the acceptance portion in the capsule endoscope system of FIG. 23.
FIG. 25 is a view illustrating an exemplified variation of the capsule endoscope system or a partial cutaway view of a system in which a connection portion of connecting the catheter tube to the wireless imaging capsule is composed of a high-molecular absorbent and an acceptance portion.
FIG. 26 is a partial cutaway view illustrating a state that the high-molecular absorbent is engaged with the acceptance portion in the capsule endoscope system of FIG. 25.
FIG. 27 is a view illustrating an exemplified variation of the capsule endoscope system or a partial cutaway view of a system in which a connection portion of connecting the catheter tube to the wireless imaging capsule is composed of a coil and an acceptance portion.
FIG. 28 is a partial cutaway view illustrating a state in which a coil is engaged with the acceptance portion in the capsule endoscope system of FIG. 27.
FIG. 29 is a view illustrating an exemplified variation of the capsule endoscope system or a partial cutaway view of a system in which a connection portion of connecting the catheter tube to the wireless imaging capsule is composed of a snap portion and an acceptance portion.
FIG. 30 is a partial cutaway view illustrating a state in which the snap portion is hooked onto the acceptance portion in the capsule endoscope system of FIG. 29.
FIG. 31 is a view illustrating an exemplified variation of the capsule endoscope system or a perspective view of a system in which a connection portion of connecting the catheter tube to the wireless imaging capsule is composed of an electro-magnet and a magnetic body.
FIG. 32 is a side sectional view illustrating the wireless imaging capsule and the catheter tube in the capsule endoscope system of FIG. 31.
FIG. 33 is a view illustrating an exemplified variation capsule of the endoscope system or a perspective view of a system in which a connection portion of connecting the catheter tube to the wireless imaging capsule is composed of two permanent magnets.
FIG. 34 is a side sectional view illustrating the wireless imaging capsule and the catheter tube in the capsule endoscope system of FIG. 33.
FIG. 35 is a view illustrating an exemplified variation of the capsule endoscope system or a sectional view of a system in which a connection portion of connecting the catheter tube to the wireless imaging capsule is a suction disc mounted at the distal end of the catheter tube.
FIG. 36 is a perspective view illustrating the wireless imaging capsule and the catheter tube in the capsule endoscope system of FIG. 35.
FIG. 37 is a sectional view illustrating a state that the suction disc of the catheter tube is adhered on the wireless imaging capsule in the capsule endoscope system of FIG. 35.
FIG. 38 is a view illustrating an exemplified variation of the capsule endoscope system or a sectional view of a system in which a connection portion of connecting the catheter tube with the wireless imaging capsule is a suction disc mounted at the distal end of the catheter tube.
FIG. 39 is a sectional view illustrating a state that the suction disc of the catheter tube is adhered on the wireless imaging capsule in the capsule endoscope system of FIG. 38.

### DESCRIPTION OF THE REFERENCE SYMBOLS

10, 20, 30, 40, 50, 60: CAPSULE LUMEN CONNECTOR
12: BALLOON
14: ACCEPTANCE PORTION
22: HIGH-MOLECULAR ABSORBENT
32: COIL
42: SNAP PORTION
52: ELECTRO-MAGNET
54: MAGNETIC BODY
62, 64: PERMANENT MAGNET
72, 74, 76: LUMEN,
73, 75, 77: OPENING
78: SUCTION DISC
80: TUBULAR PORTION
82: PROJECTION
84: INTERNAL LUMEN
85: PROXIMAL OPENING
90: TUBULAR PORTION
100, 100A TO 100H: WIRELESS IMAGING CAPSULE
102: STRING TETHER
104, 104', 104A TO 104H: CATHETER TUBE
106: ESOPHAGUS
108: STOMACH
110: LOWER ESOPHAGEAL SPHINCTER
112: UPPER ESOPHAGEAL SPHINCTER
114: ORAL CAVITY
116: TONGUE
130: OVERTUBE
134: PACKING
152: CENTRAL LUMEN
154: CHANNEL
176: DILATION TUBE
178: SLIT
200: INTRAPERITONEAL CAVITY
201: MARKING
202: ANTERIOR ABDOMINAL WALL
204: TROCAR
206: SURGICAL INSTRUMENT
208: INTESTINE
202: HANDLE
250: GRASPING FORCEPS
250: LAPAROSCOPE
260: ENDOSCOPE
262: CAPSULE HOLDER
264: INCISION
300: MOUTHPIECE
302: TEETH
502: OBJECTIVE LENS
404: INTERNAL LUMEN
406: CAPSULE LUMEN CONNECTOR
700: CONTROLLER

### BEST MODE FOR CARRYING OUT THE INVENTION

Examination of the patient's esophagus is performed in the following general manner. The patient is prepped by applying external RF antennas to the patient's skin in an area that is conducive to receiving signals transmitted by a capsule endoscope. The antennas are connected to a recording device such as a portable recorder worn around the patient's waist which can store the images for future display, or ideally to a device which produces a live video display of the images captured by the capsule endoscope. Prior art has reported that tethered capsule endoscope exams are hindered if the procedure is performed "blindly" by first recording the images during the maneuvers of the exam and then reviewing the images only after the exam has been completed. A real-time view of the images captured by the capsule endoscope will allow the operator to effectively use the air, water and suction features of the present invention to obtain the best images of the anatomy possible. Real-time display of the images captured by the capsule endoscope can be obtained by using a dedicated viewer designed for this function or a computer equipped with hardware and software to capture and display images obtained from the capsule endoscope in real time.

After prepping the patient and setting up the equipment, the patient places the capsule endoscope in his/her mouth with the string tether loosely positioned to follow the capsule endoscope. The patient then swallows the capsule endoscope with a small amount of water (a so-called wet swallow) to facilitate movement of the capsule endoscope into the esophagus. Repeated small wet swallows may be necessary to pass the capsule endoscope into the stomach. The operator can determine that the capsule endoscope has entered the stomach by checking a mark on the string tether (Typically a 50 cmmark indicates position in the stomach.) Once the capsule endoscope is in the stomach, the string tether is gently pulled to bring the capsule endoscope to the lower esophageal sphincter (LES), which can be determined by the additional mild resistance felt when pulling on the string tether. At this point, a catheter tube is gently advanced over the string tether until the distal end of the catheter tube impinges on the proximal end of the capsule endoscope. The string and the tapered opening in the back of the capsule endoscope together guide the distal end of the catheter tube into the back end of the capsule. This connects the lumen of the catheter tube with the internal lumen of the capsule endoscope. A proper connection can be confirmed by injecting water through the catheter tube and observing via the real-time viewer that it squirts out of the end of the capsule.

One embodiment of the present invention has a controller that allows the operator to independently control the flow of air, water and fluids to and from the capsule endoscope. This embodiment has both an air source, for example an air pump, and a suction source such as a suction pump. The suction source is connected to a suction valve that controls the application of suction to the catheter tube and ultimately the internal lumen of the capsule endoscope. The air source is likewise connected to an air valve that controls the injection of air through the catheter tube and ultimately the internal lumen of the capsule endoscope. The air source also pressurizes a water-filled container to force water out of the container to a water valve. The water valve controls the injection of water through the catheter tube and ultimately the internal lumen of the capsule endoscope.

After proper connection of the catheter to the capsule endoscope is confirmed by feeding and observing the flow of water from the capsule, the patient is asked to swallow a sip of water to open the LES while the operator gently pulls on the catheter, allowing the capsule to slip back up into the lower esophagus. From here the capsule is slowly pulled up the esophagus while observing the images on the real time viewer until a slight resistance indicates that the capsule has reached the UES. The patient is then instructed to make small wet swallows moving the capsule down the esophagus once again. Slight resistance can be placed on the catheter to maintain its position in the esophagus for extended observation.

At any point that bubbles are observed obscuring the image, the operator can suction and remove these offending bubbles by operating the suction valve. Likewise, salvia obscuring the image can be washed away or suctioned away by operating the water or suction valves respectively. A good view of the distal esophagus is facilitated by injecting air or water to distend the tissue around the LES. This is accomplished by operating the air and water valves, respectively.

If sufficient observation of the esophagus is not possible in a single pass, once the capsule endoscope passes through the LES and into the stomach, the entire process can be repeated again by pulling the capsule backup into the esophagus and studying the esophagus for a second (or third) time. Images of diseased areas are recorded by the imaging system for subsequent documentation purposes. Furthermore, the marks on the catheter tube can be used to measure the approximate length of any observed diseased area (e.g., Barrett's esophagus).

After the esophagus is sufficiently examined, gentle retraction of the catheter brings the capsule endoscope to the proximal end of the esophagus where a slight increase of resistance indicates that it is at the level of the UES. The patient is then instructed to make a dry swallow while the catheter is pulled by the catheter into and out of the patient's mouth.

An alternate method of use of the present invention is to insert the custom mouthpiece into the patient once the capsule endoscope has been initially swallowed. One embodiment of the mouthpiece has a cutout running the length of the mouthpiece, allowing the mouthpiece to be slid over the string tether from its side. Once over the string, the mouthpiece is inserted into the patient's oropharynx and held in place by gentle pressure from the patient's teeth. The mouth piece and its extension over the tongue of the patient facilitates holding the string tether and the catheter in the midline of the patient, and reduces the discomfort of the presence of the string and catheter in the patient's mouth.

An alternate method for retrieving the capsule endoscope in the present invention is to apply a dilation catheter over the catheter tube to minimize the size transition between the capsule endoscope and the catheter and provide a more gradual transition when pulling the capsule endoscope through tight areas of the patient's anatomy upon retrieval.

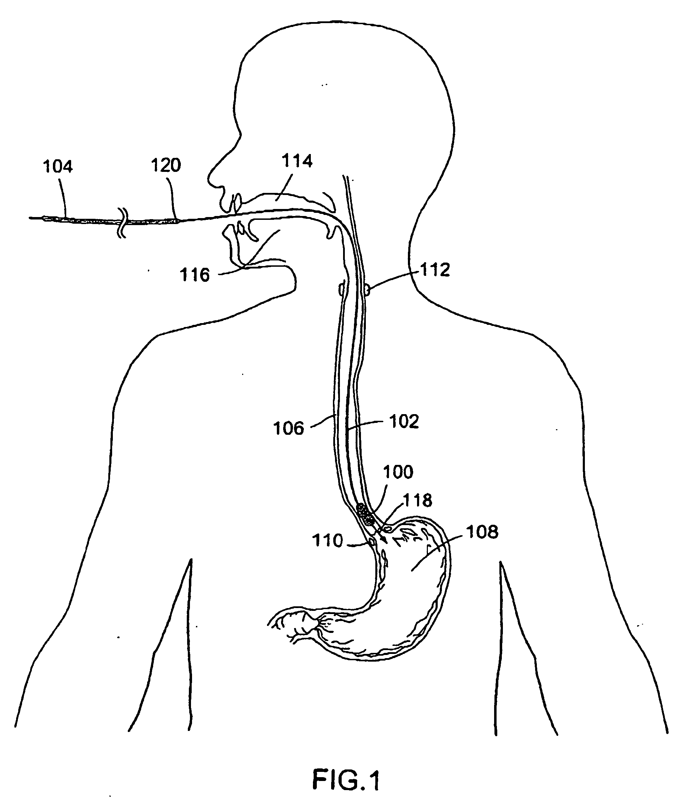

FIG. 1 schematically illustrates the wireless imaging capsule (capsule endoscope) 100 in use in the application of observing a patient's esophagus. The anterior sectional view of the patient illustrates the relative positions of the oral cavity 114, the tongue 116, the esophagus 106, and the stomach 108. At its proximal end, the esophagus is bounded by the upper esophageal sphincter (UES) 112. At its distal end, the esophagus is bounded by the lower esophageal sphincter (LES) 110.

As illustrated in FIG. 1, the patient has swallowed a wireless imaging capsule 100 which is connected to the distal end of a thin, flexible string tether 102. The proximal end of the string tether 102 exits the patient's mouth. The string tether 102 is passed through a lumen 408 of a catheter tube 104. The distal end 120 of a catheter tube 104 is positioned over the string tether 102 ready for insertion into the patient.

The wireless imaging capsule 100 contains an imaging system which images the interior of the patient's body with a direction of view 118 that is approximately coincident with the axis of the wireless imaging capsule 100: The entire length of the wall of the esophagus 106 can be imaged as the wireless imaging capsule 100 moves from one end of the esophagus to the other. The wireless imaging capsule 100 transmits the images it obtains by radio frequency transmissions to external antennas and a recording and display device (not shown). This technology and these devices are common in the industry.

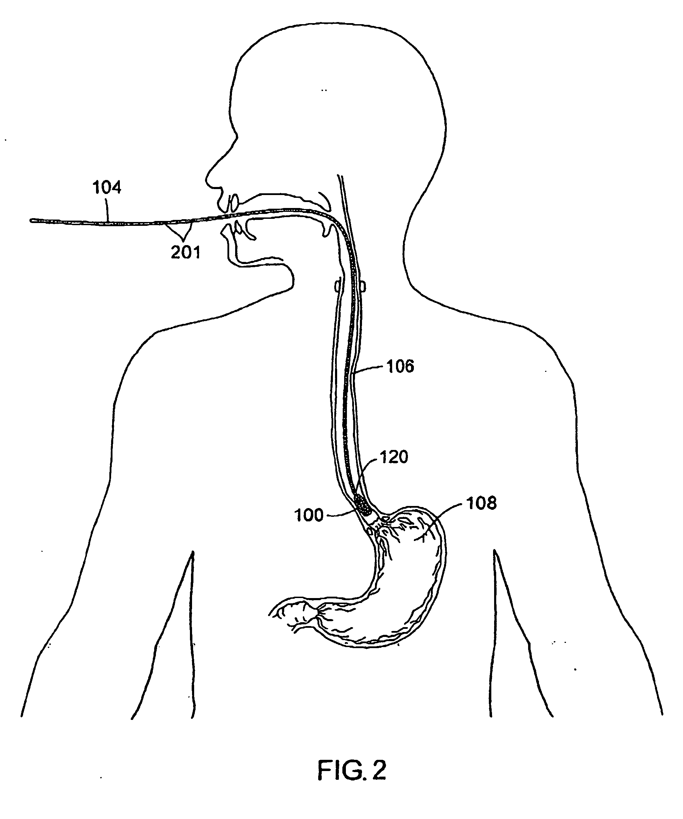

FIG. 2 illustrates that after the patient swallows the wireless imaging capsule 100, the catheter tube 104 is advanced over the string tether 102 until the distal end 120 of the catheter tube 104 connects with the proximal end of the wireless imaging capsule 100. Markings 201 on the external surface of the catheter tube 104 enable the operator to determine how far the wireless imaging capsule 100 is in the body and are also useful for determining the position and measuring the length of any lesions found (e.g., measuring the length of a segment of Barrett's esophagus).

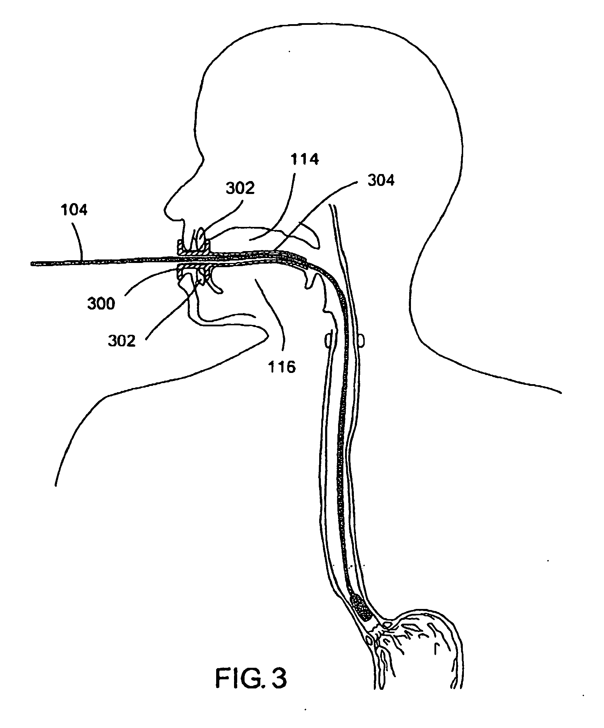

FIG. 3 illustrates an embodiment of the present invention employing a mouthpiece 300 that has been inserted into the patient's oral cavity 114 and is held in place by the patient's teeth 302 as the patient gently bites on the mouthpiece. A projection 304 on the mouthpiece extends into the patient's oral cavity 114 and guides the catheter tube 104 into the midline of the oral cavity and over the patient's tongue 116. The mouthpiece improves the patient's comfort in having the catheter tube 104 in the mouth and reduces the sensation of movement as the operator manipulates the catheter tube 104 during the examination.

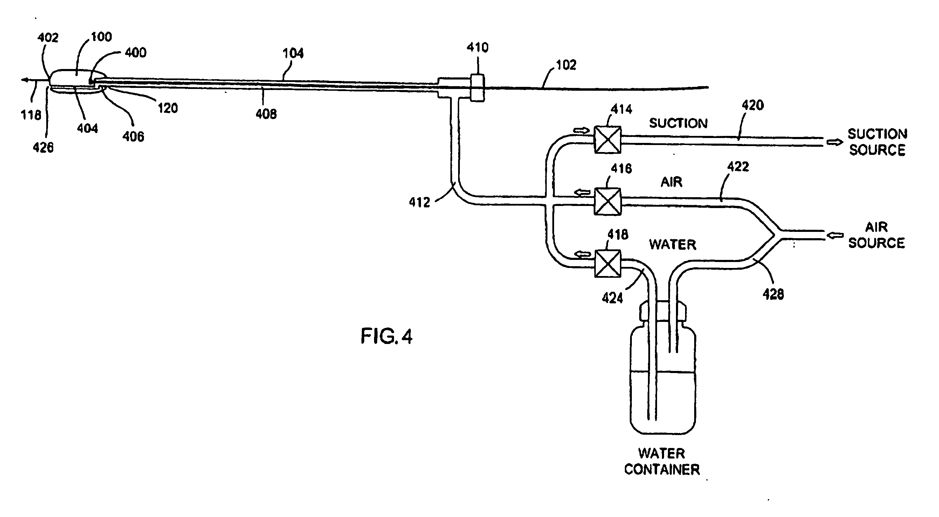

FIG. 4 is a schematic of the various components of a first embodiment of the present invention. The wireless imaging capsule 100 has a string tether 102 attached to its proximal end at a fixation point 400. A vision system incorporated into the wireless imaging capsule 100 (not shown in FIG. 4, but using standard technology) views through an objective lens 402 at the distal end of the capsule with a direction of view 118 coincident with a longitudinal axis of the wireless imaging capsule 100. The wireless imaging capsule 100 has an internal lumen 404 running between the proximal and distal ends of the capsule 100. This lumen is for conveying air, water and fluids through the capsule.

FIG. 4 illustrates the configuration of the capsule after it has been swallowed and the catheter tube 104 has been advanced over the string tether 102 and the distal end 120 of the catheter tube 104 has mated with the proximal end of the capsule 100 at the connection point 406 between the capsule and catheter. When the catheter tube 104 and capsule 100 are thus connected, the lumen 408 of the catheter tube 104 and the inner lumen 404 of the wireless imaging capsule 100 are joined so that they form a single channel for the flow of gas and fluids. The wireless imaging capsule 100 and catheter tube 104 are initially pulled together by the string tether 102 and are moderately held together by friction at the capsule lumen connector 406 between the two devices. Additionally, the string tether 102 is pulled taut and held relative to the catheter tube 104 by a string fixation device 410 at the proximal end of the catheter tube 104.

As FIG. 4 illustrates, an air, water and suction system is attached to the catheter tube 104 via a supply tube 412. The application of suction, air and water to the supply tube 412 is controlled by a suction valve 414, air valve 416 and water valve 418 respectively. A suction tube 420 connects the suction valve 414 to an appropriate source of suction such as a portable medical suction pump, or a hospital facility suction system. An air tube 422 connects the air valve 416 to a source of air flow such as a portable medical air pump (e.g., the type used for endoscopy equipment).

While roller pumps and other types of water pumps are commonly used as a source of water flow for medical equipment, a common method of supplying water to endoscopic equipment is to pressurize a container of water using pressurized air, thus forcing water out of the container. As FIG. 4 illustrates, in this embodiment of the present invention, the water container is pressurized by connecting it to the air source by the water container tube 428. The pressure forces water out of the water container through a water tube 424 which is connected to the water valve 418. The suction valve 414, air valve 416 and water valve 418 are in a normally-closed position such that all flow to and from the supply tube 412 is restricted when the valves are not operated. Opening the suction valve 414 will suction any fluid or gas (e.g., air) present at the open distal end 426 of the capsule's internal lumen 404 to the suction source. Opening the air valve 416 will allow air to flow from the air source through the air tube 422, the supply tube 412, the catheter tube 104, the capsule inner lumen 404 and out the open distal end 426 of the capsule lumen. Opening the water valve 418 will allow water from the water container to flow through the water tube 424, the supply tube 412, the catheter tube 104, the capsule inner lumen 404 and out the open distal end 426 of the capsule lumen.

During the patient's examination, the operator will operate the air, water and suction valves as needed to wash the area in front of the objective lens 402, to suction fluids and bodily secretions (e.g., saliva), and to insufflate air to expand the organ under observation. These functions impinge on the tissues directly ahead of the capsule within its direction of view 118.

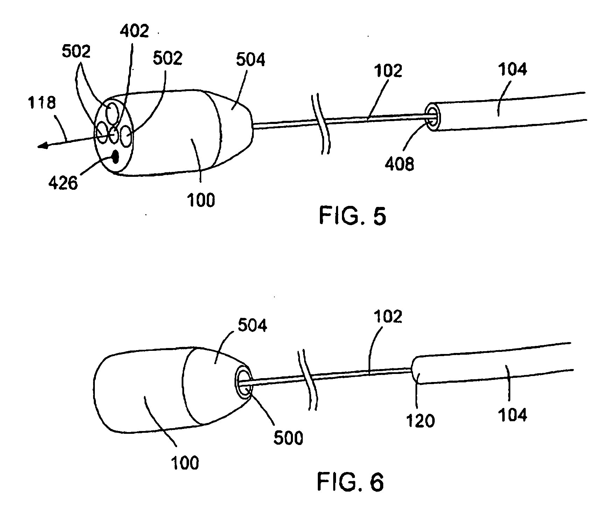

FIG. 5 illustrates one embodiment of the wireless imaging capsule 100. The wireless imaging capsule 100 has an objective lens 402 on its distal end which obtains images of the GI tract with a direction of view 118 approximately parallel to the axis of the capsule. One or more light-emitting diodes (LEDs) 502 or other suitable light source provides internal illumination of the GI tract. The wireless imaging capsule 100 is that it contains an internal lumen which has a distal opening 426 on the distal end of the capsule and a proximal opening that ends in a capsule lumen connector 500, as illustrated in FIG. 6. This capsule lumen connector enables coupling of the internal lumen 408 of the catheter tube 104 to the internal lumen of the wireless capsule. The catheter tube 104 is guided over the string tether 102 until the distal end 120 of the catheter tube 104 fits snugly into the capsule lumen connector 500.

FIG. 5 and FIG. 6 also illustrate that in this embodiment of the present invention, the proximal end of the wireless imaging capsule 100 is tapered 504. The function of this taper is to facilitate the passage of the capsule in a retrograde fashion through narrow areas of the patient's anatomy, such as the LES and UES as the capsule is pulled backwards by the tether . Reports in the medical literature indicate that the spherical shape found on prior art capsules is difficult to pull through this narrow areas of the patient's anatomy.

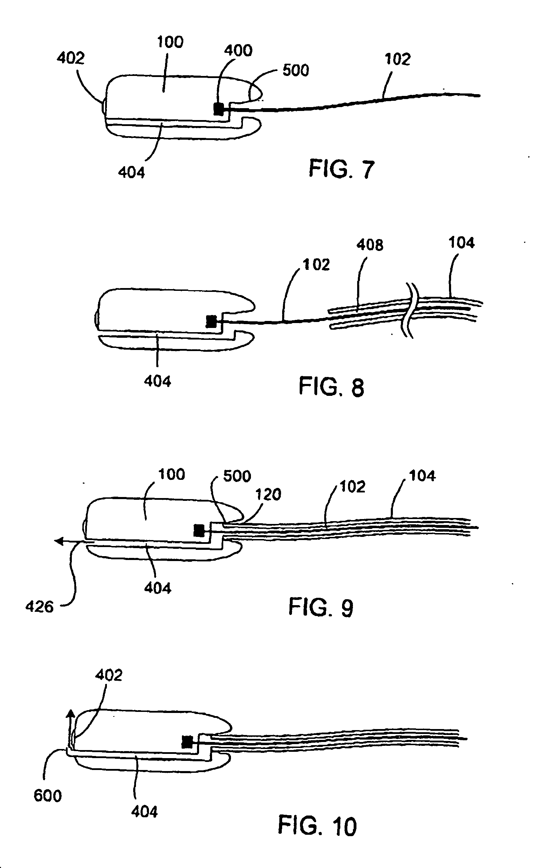

FIG. 7 through FIG. 9 illustrate the process of connecting the catheter tube 104 to the wireless imaging capsule 100. FIG. 7 illustrates that the fixation point 400 of the string tether 102 is such that it is positioned to bring the distal end of the string tether 102 into the approximate center of the capsule lumen connector 500. FIG. 8 illustrates the advancement of the catheter tube 104 over the string tether 102. FIG. 9 illustrates that the string tether 102 guides the distal end 120 of the catheter tube 104 into the capsule lumen connector 500. A friction fit between the capsule lumen connector 500 and the distal end 120 of the catheter tube 104 creates a seal between the wireless imaging capsule 100 and the catheter tube 104. This seal ensures that gas or fluids injected into the lumen 408 of the catheter tube 104 will pass through the inner lumen 404 of the wireless imaging capsule 100 and out of the distal opening 426 of this lumen.

FIG. 10 illustrates an alternate embodiment of the present invention. In this embodiment a nozzle 600 on the distal end of the inner lumen 404 directs the flow of liquids and gas passing through the inner lumen over the surface of the objective lens 402, enabling the lens to be washed with fluid and any remaining water drops to be blown off with air.

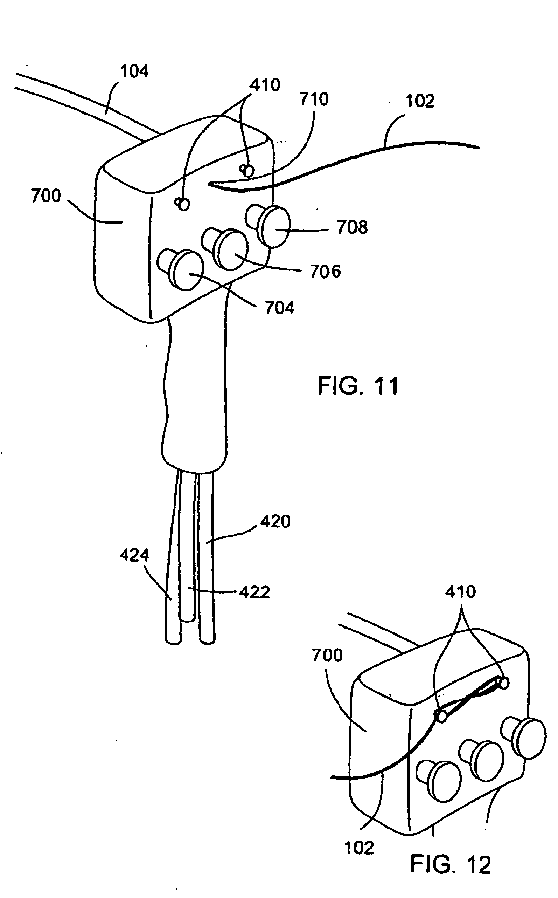

FIG. 11 illustrates an embodiment of a controller for the air, water and suction system schematically illustrated in FIG. 4. The controller 700 houses the air, water and suction valves shown in FIG. 4. The controller is designed to be held in the operator's hand by gripping the handle 702 of the controller. The operator's thumb is then able to operate the water valve button 704, the air valve button 706 and the suction valve button 708. A water tube 424, an air tube 422 and a suction tube 420 enter the handle 702 of the controller and bring water, air and suction to the internal water, air and suction valves, respectively. The catheter tube 104 also connects to the controller and brings water, air and suction to the wireless capsule at it distal end (not shown). The string tether 102 travels through the inner lumen of the catheter tube 104, enters the controller and then exits through an opening 710 in the controller. There are many means of holding the string 102 tether taut within the lumen of the catheter tube 104. Those skilled in the art can design a variety of means of attachment using compression rings, friction devices, knotted devices, etc. without exceeding the spirit and scope of the present invention. In the embodiment illustrated in FIG. 11, the string tether fixation device 410 consists of two simple posts. The operator tensions the string tether 102 and then wraps the string tether 102 around the posts as illustrated in FIG. 12.

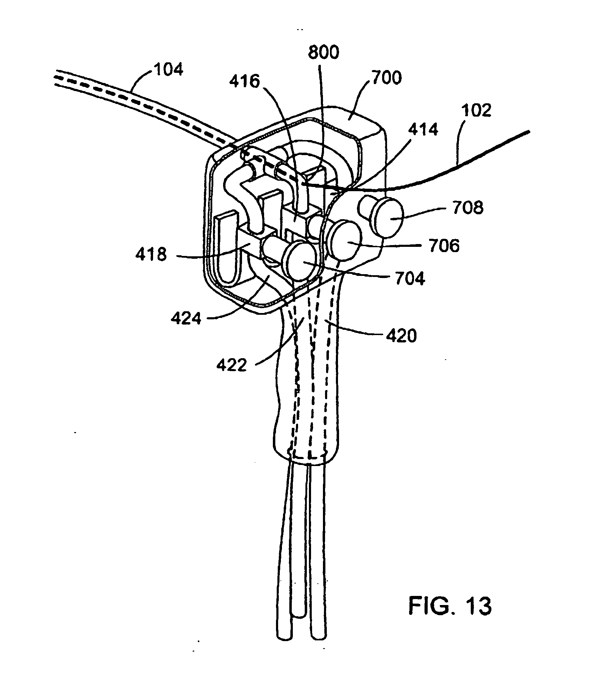

FIG. 13 is a cut-away illustration of the controller illustrated in FIG. 11. In this embodiment the water tube 424, the air tube 422 and the suction tube 420 pass through the water valve 418, the air valve 416 and the suction valve 414 respectively. These valves control the passage of air and fluids in and out of the catheter tube 104. FIG. 13 also illustrates that in this embodiment of the present invention, the string tether 102 which travels through the inner lumen of the catheter tube 104 exits the supply tubing system through a tight puncture 800 in the wall of the tubing, and then through the string opening 710 in the controller as shown in FIG. 11. This tight puncture in the wall of the plastic tubing used to construct the air, water and suction feeding system allows the string tether to be pulled at its proximal end to advance the catheter tube 104 over the string tether 102 until it mates with the wireless imaging capsule 100, while at the same time preventing the leakage of air or fluids around the string tether 102 at the puncture. Those skilled in the art can design alternate means of sealing around the string tether without exceeding the spirit and scope of the present invention.

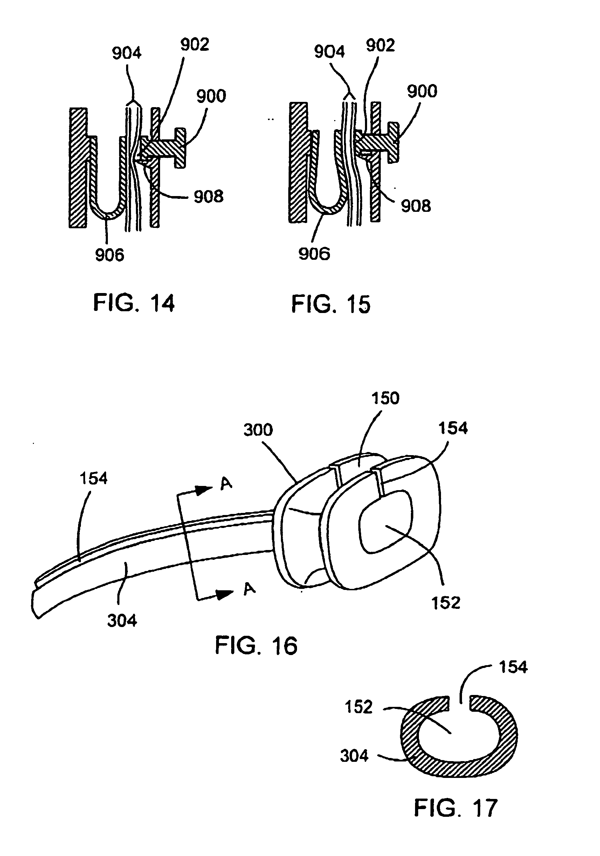

FIG.14 and FIG. 15 provide further cross-sectional detail of the valves illustrated in FIG. 13. In this embodiment, the valve has several major components - a button 900 by which the operator can press on the valve to open it, a hole 902 in the valve stem through which a section of plastic tubing 904 passes, and a spring member 906. In the non-activated condition (FIG. 14) the spring member forces the wall of the tubing against a fixed projecting edge 908. The wall of the tubing is sufficiently pliable to allow it to collapse under the spring pressure against this protruding edge, thereby occluding the lumen of the tubing and preventing flow through the tubing.

FIG. 15 illustrates that when the operator depresses the valve button 900, the spring member 906 further compresses bringing the hole 902 in the valve stem beyond the extent of the fixed projecting edge 908 preventing it from compressing the wall of the tubing 904 passing through the hole. The inherent springiness in the wall of the tubing causes it to open, thereby allowing air and fluids to flow through the tube. The advantages of this embodiment is that the valve is of an inexpensive, one-piece construction, the valve is normally closed preventing flow, it is easily opened by the operator by depressing the button, and it springs back to its normally closed position when the operator removes his/her thumb from the valve button.

The embodiments illustrated in FIG. 5 through FIG. 15 are purposefully designed to reduce their manufacturing cost in order to allow the devices to be marketed as single-use disposable devices. Based on the teaching of this patent, those skilled in the art can design alternate embodiments that have enhanced complexity and higher intended durability and designed for easy disassembly for cleaning and disinfection prior to reuse on subsequent patients. In particular many alternate designs for the air, water and suction valves, for the means of coupling the end of the catheter tube to the capsule and the means for fixing the string tether with respect to proximal end of the catheter tube can be conceived without exceeding the scope and spirit of the present invention.

FIG. 16 illustrates one embodiment of the mouthpiece 300 illustrated in FIG. 3. The mouthpiece has a depression 150 in its proximal end that enables the patient to gently bite down on the mouthpiece to hold it comfortably in the mouth. The shape of the depression is such that the patient' s teeth will comfortably and securely fit into the depression. The distal end of the mouthpiece has a projecting part 304 with a shape and length that allows it to fit comfortably over the patient's tongue. A central lumen 152 runs through the mouthpiece from its proximal to its distal end. A channel 154 is also cut through the entire wall of the mouthpiece from its proximal to its distal end. This channel enables the mouthpiece to be slid over the catheter tube from the side of the catheter tube. FIG. 17 is a cross-section of the mouthpiece projecting part 304 illustrating its central lumen 152 and the channel 154 cut into its wall.

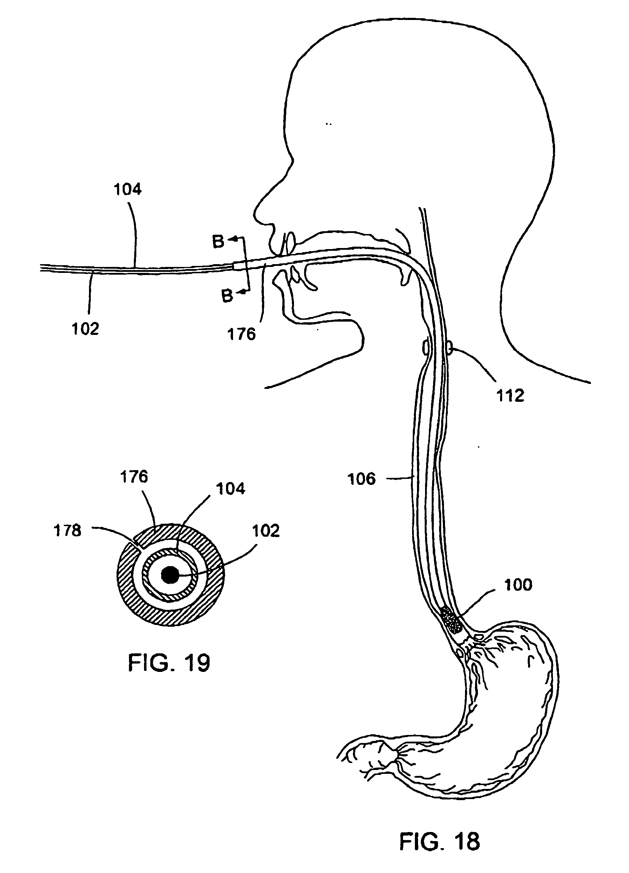

FIG. 18 is an illustration of the use of a dilation tube 176 to facilitate removal of the wireless imaging capsule 100 from the patient. After completion of the examination, the wireless imaging capsule 100 must be withdrawn from the patient by pulling on the string tether 102. Prior experience with tethered capsules indicates that retrograde withdrawal through tight areas of the patient's anatomy such as the UES 112 is difficult due to the size differential between the small diameter of the tether and the large diameter of the capsule. In addition to smoothing this transition by tapering the proximal end of the capsule as illustrated in FIG. 5 and FIG. 6, FIG. 18 illustrates an embodiment of the present invention using a dilation tube. The dilation tube 176 is long enough to reach at least from the patient's teeth to the lower esophagus. As illustrated in FIG. 19, the dilation tube 176 has a slit 178 in its wall allowing the pliable wall of the dilation tube to be spread apart so that it can be slid sideways over the length of the catheter tube 104 that extends from the patient's mouth. Once the dilation tube is completely encapsulating the catheter tube 104 throughout the dilation tube's length, the dilation tube is gently advanced over the catheter tube 104 until the distal end of the dilation tube abuts the proximal end of the wireless imaging capsule 100, as illustrated in FIG. 18. After placing in this position, the operator withdraws the string tether 102, the catheter tube 104 the dilation tube 176 and the wireless imaging capsule 100 as a unit. The smooth transition in size between the diameter of the dilation tube and the wireless imaging capsule 100 facilitate retrograde withdrawal of the wireless imaging capsule 100 through tight areas of the patient's anatomy such as the UES 112.

While the embodiments of the present invention are shown to be appropriate for use within the esophagus of the patient, the same methods and apparatus can be used to examine other lumens of the body - in particular, the stomach, the small intestine and the colon. For these applications an adjustment in the length of the string tether, the catheter tube and the dilation tube will allow the wireless imaging capsule to be inserted into areas of the body which are more remote from the point of access (i. e. , from the patient's mouth, from the patient's anus, etc.).

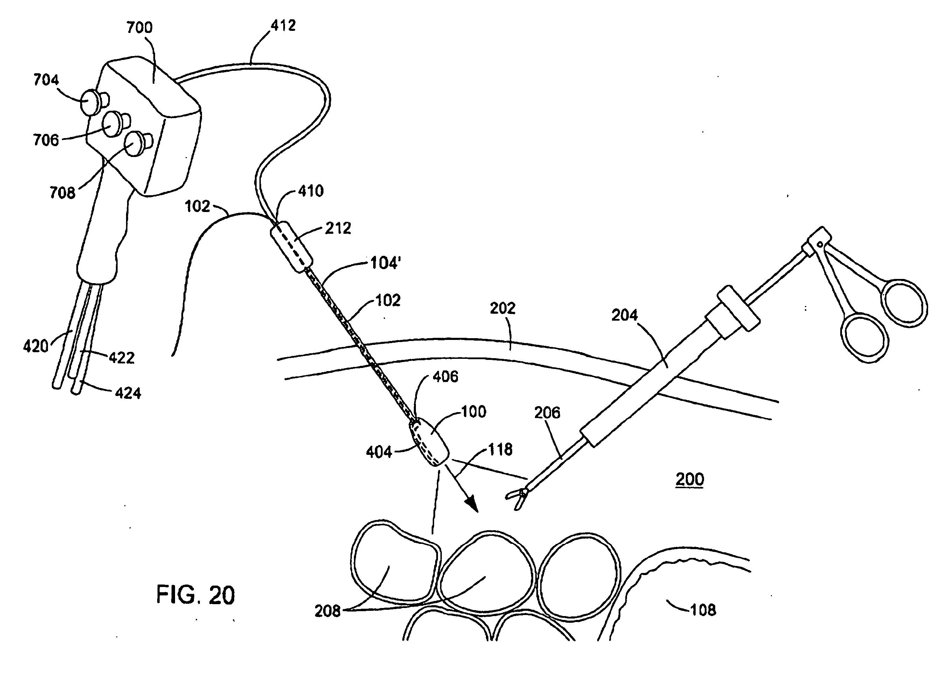

FIG. 20 illustrates the application of the present invention in intraperitoneal endoscopy (laparoscopy). In this embodiment the wireless imaging capsule 100 is held on the end of a thin rigid catheter tube 104' which is in turn connected to a supply tube 412, which in turn is connected to a controller 700. The wireless imaging capsule 100 has an internal lumen 404 for conveying gas and fluids through the capsule. The controller is connected to sources of air, water, and suction (sources not shown) via an air tube 422, a water tube 424 and a suction tube 420. Air 706, water 704 and suction 708 buttons on the controller feed air and water and apply suction to the inner lumen 404 of the wireless imaging capsule 100, respectively. It is common practice in endoscopy of the peritoneal cavity to use gases other than air for insufflating the intraperitoneal cavity 200. Therefore, the commonly used gases of carbon dioxide or nitrous oxide may be used as a substitute for air in this embodiment. In this embodiment it is preferable to use a rigid catheter tube rather than the flexible catheter tube used in the embodiment designed for examining the esophagus. The rigid catheter tube 104' allows the operator to easily control the position and movement of the wireless imaging capsule 100 in the intraperitoneal cavity by manipulating the catheter tube 104' and its handle 212. The wireless imaging capsule 100 is connected to the catheter tube 104' by a connection 406 which interconnects the inner lumen of the capsule 404 with the lumen of the catheter tube 104'.

In this application the wireless imaging capsule 100 is useful for observing and guiding the use of a variety of surgical instruments 206 introduced into the intraperitoneal cavity 200 through punctures in the anterior abdominal wall 202. These surgical instruments 206 are typically inserted through trocars 204 which provide easy access and easy exchange of instrumentation brought into the intraperitoneal cavity. Images obtained by the wireless imaging capsule 100 with a direction of view 118 along the axis of the wireless imaging capsule 100 are used to guide surgery of the intestines 208, the stomach 108 and other peritoneal organs.

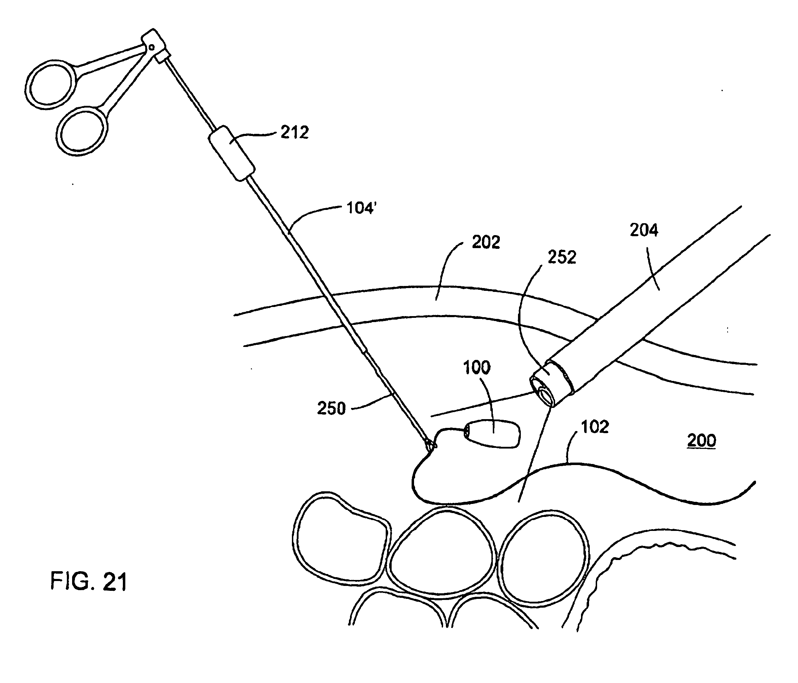

Prior to using the wireless imaging capsule 100 as configured in FIG. 20, the wireless imaging capsule 100 must be introduced into the intraperitoneal cavity. One method of doing this is by means of the apparatus illustrated in FIG. 21. As a first step, a large trocar 204 is placed through the abdominal wall 202 of the patient for access to the intraperitoneal cavity 200. The wireless imaging capsule 100 with its attached string tether 102 is then slid through the open trocar tube 204 into the intraperitoneal cavity. A laparoscope 252 is then introduced through the trocar to provide a means of seeing the capsule in the intraperitoneal cavity. The rigid catheter tube 104' is than placed through the abdominal wall 202 (perhaps with the assistance of an obturator - not shown but standard in the art). Through the lumen of the catheter tube 104' a thin grasping forceps 250 is inserted into the intraperitoneal cavity. Guided by the image system of the laparoscope 252, the grasping forceps is maneuvered to pick up the string tether 102 and to pull it through the lumen of the catheter tube 104', bringing the proximal end of the string tether 102 outside the patient, exiting through the handle 212 of the catheter tube 104'. By pulling on the string tether 102 the operator then pulls the proximal end of the capsule to the distal end of the catheter tube 104' and connects the two devices together, as described for the prior embodiments. At this point the supply tube 412 can be connected to the handle 212 and the rest of the system configured as illustrated in FIG. 20.

Although a thin grasping forceps 250 is shown as the means of capturing and bringing the string tether 1.02 out of the body, other embodiments of a grasping means are well known. These include a variety of hooks, snares and other thread capture tools.

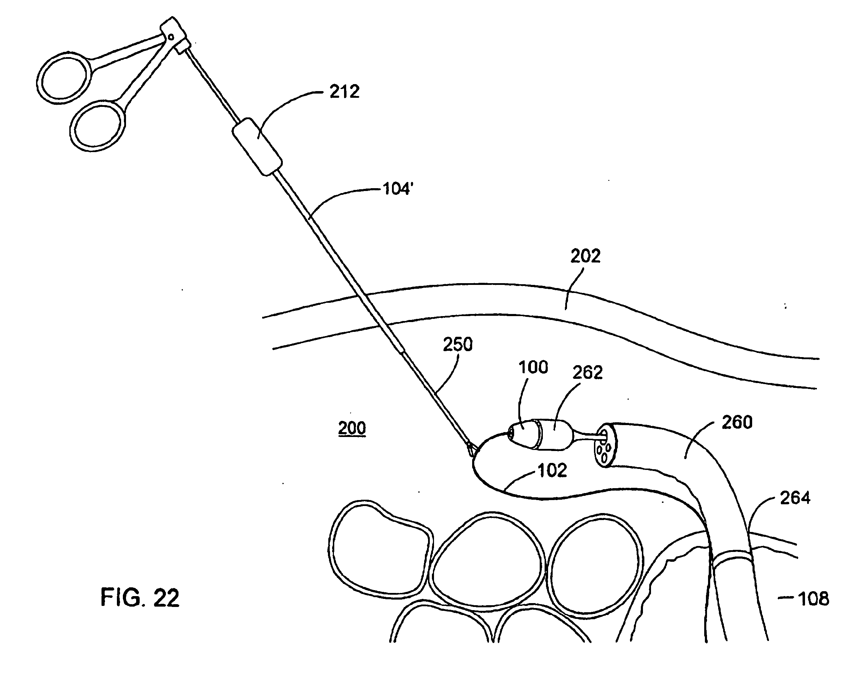

FIG. 22 illustrates another means of bringing the imaging capsule into the intraperitoneal cavity. This method employs the use of a flexible endoscope passed thorough the patient's mouth into the stomach 108. An incision 264 in the wall of the stomach allows the tip of the endoscope 260 to leave the stomach and enter the intraperitoneal cavity 200. A capsule holder 262 passed through the endoscope channel brings the wireless imaging capsule 100 and its string tether 102 into the intraperitoneal cavity 200. As in FIG. 21, a thin grasping forceps 250 inserted through the catheter tube 104' captures the string tether 102 and pulls it through the lumen of the catheter tube 104'. Once the string tether 102 is outside the body, it is pulled to bring the proximal end of the capsule to the catheter tube 104', joining the two devices together. The rest of the equipment can then be assembled in the configuration shown in FIG. 20.

Although FIG. 22 illustrates access to the intraperitoneal cavity through an incision in the wall of the stomach, the tip of the flexible endoscope can be brought into this same space by passing the endoscope through the anus and then making an incision in the wall of the colon, in order to gain entry in the intraperitoneal cavity.

When a wireless imaging capsule 100 is retrieved, a catheter tube 104' may be separated from the wireless imaging capsule 100 to retrieve the wireless imaging capsule 100 through a trocar 204, or a flexible endoscope placed into the stomach 108 may be used to retrieve the same. This eliminates the necessity for widening an opening of the abdominal wall through which the catheter tube 104' is passed to a dimension large enough for allowing the wireless imaging capsule 100 to pass through. Therefore, capsule retrieval is facilitated to relieve a patient of unnecessary burden.

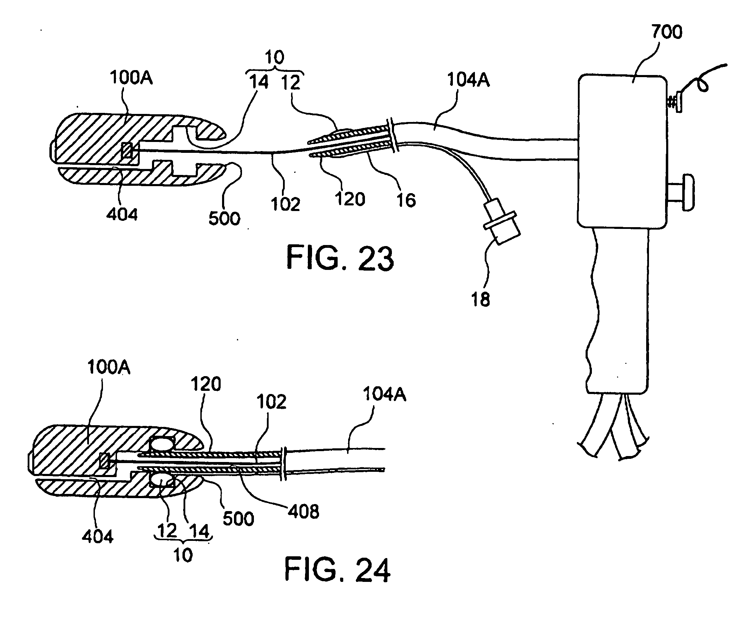

A description will be made for other embodiments of the capsule endoscope system in the present invention.
The capsule endoscope system of the present invention in FIG. 23 and FIG. 24 is provided with a capsule lumen connector (connection portion) 10 for connecting the distal end 120 of the catheter tube 104A to the wireless imaging capsule 100A. As illustrated in FIG. 23, the capsule lumen connector 10 of the present embodiment is provided with a balloon (dilation member) 12 mounted on an outer peripheral surface of the distal end 120 of the catheter tube 104A and which dilates as appropriate, and an acceptance portion 14 mounted on the wireless imaging capsule 100A and which accepts the balloon 12.

Upon injection fluids such as air, gas and water, the balloon 12 inflates so as to expand the outer diameter of the distal end 120. The acceptance portion 14 is formed circumferentially as a groove on an inner face of the internal lumen 404 of the capsule. The balloon 12 inflated so as to expand the outer diameter is engaged with the groove.
The catheter tube 104A is provided with a fluid tube 16 for injecting a fluid into the balloon 12. The fluid tube 16 is fixed to the catheter tube 104A so as to run along the longitudinal direction. The distal end of the fluid tube 16 is connected to the balloon 12 and a fluid injection port 18 is provided at the proximal end.
In addition, the distal end 120 of the catheter tube 104A is tapered in such a way that the diameter thereof is made smaller as the catheter tube 104A comes closer to the leading end face.

When the distal end 120 of the catheter tube 104A is connected to the wireless imaging capsule 100A, the proximal end of the string tether 102 is at first passed through a lumen 408 of the catheter tube 104A, and then the catheter tube 104A is advanced along the string tether 102. When the distal end 120 of the catheter tube 104A is brought into contact with the wireless imagine capsule 100A, the catheter tube 104A is further advanced in such a way as to pull the string tether 102, and the distal end 120 of the catheter tube 104A is passed through a proximal opening 500 of the wireless imaging capsule 100A and inserted into the internal lumen 404. Upon insertion of the distal end 120 of the catheter tube 104A into the internal lumen 404, a syringe (not illustrated) is connected to the fluid injection port 18 to inject a fluid into a balloon 12 via the fluid tube 16. As illustrated in FIG. 24, upon injection of the fluid, the balloon 12 is inflated so as to expand the outer diameter of the distal end 120 of the catheter tube 104A and engaged with a groove-shaped acceptance portion 14. Thereby, the distal end 120 of the catheter tube 104A is connected to the wireless imaging capsule 100A so as not to be easily disengaged therefrom, and the lumen 408 of the catheter tube 104A is communicatively connected with the internal lumen 404 of the wireless imaging capsule 100A. Furthermore, the balloon 12 is firmly attached to the inner face of the acceptance portion 14, thereby sealed is a space between the distal end 120 of the catheter tube 104A and the internal lumen 404 of the wireless imaging capsule 100A.

When the distal end 120 of the catheter tube 104A is separated from the wireless imaging capsule 100A, a fluid is discharged from the balloon to shrink the balloon 12. Thereby, the distal end 120 including the balloon 12 of the catheter tube 10 4A is decreased in outer diameter and the balloon 12 is disengaged from the acceptance portion 14. Then, only the catheter tube 104A may be pulled out, by which the distal end 120 of the catheter tube 104A is separated from the wireless imaging capsule 100A.

According to the thus constituted capsule endoscope system, the catheter tube 104A can be easily connected to or disconnected from the wireless imaging capsule 100A. Furthermore, fluids such as water, air and gas can be supplied to the body of the patient via the catheter tube 104A and the wireless imaging capsule 100A, and water supplied into the body of the patient or body fluids such as saliva can be suctioned.

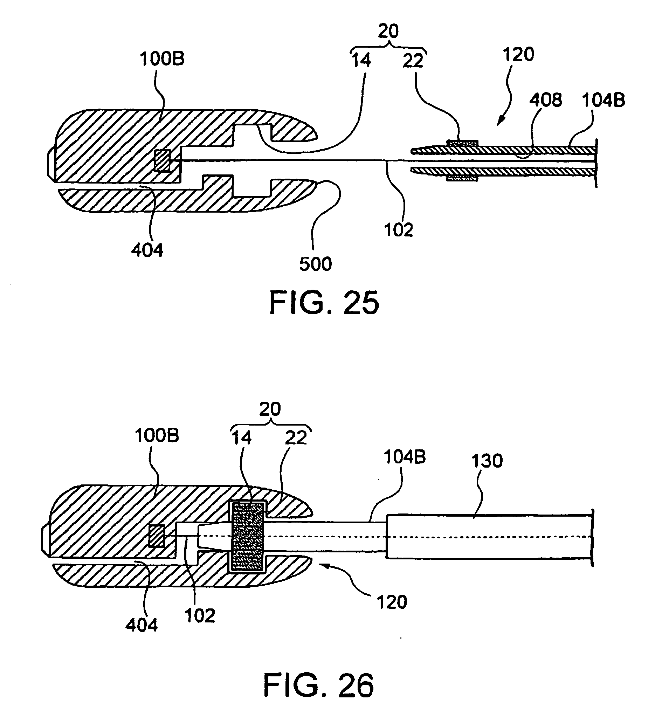

The capsule endoscope system of the present invention illustrated in FIG. 25 and FIG. 26 is provided with a capsule lumen connector (connection portion) 20. As illustrated in FIG. 25, the capsule lumen connector 20 of the present embodiment is provided with a high-molecular absorbent (dilation member) 22 such as cellulose mounted on the outer peripheral face of the distal end 120 of the catheter tube 104B and an acceptance portion 14.

Upon supply of a liquid such as water, the high-molecular absorbent 22 is dilated so as to expand the outer diameter of the distal end 120. The high-molecular absorbent 22 dilated so as to expand the outer diameter is engaged with a groove-shaped acceptance portion 14.
In addition, the distal end 120 of the catheter tube 104B is tapered so that the diameter thereof is decreased as the distal end comes closer to the leading end face of the catheter tube 104B.

When the distal end 120 of the catheter tube 104B is connected to the wireless imaging capsule 100B, as described above, the distal end 120 of the catheter tube 104B is passed through a proximal opening 500 of the wireless imaging capsule 100B and inserted into an internal lumen 404. Then, a liquid such as water is supplied via the catheter tube 104B to the internal lumen 404 of the wireless imaging capsule 100B. Upon supply of the liquid to the internal lumen 404, as illustrated in FIG. 26, the high-molecular absorbent 22 is dilated so as to expand the outer diameter of the distal end 120 of the catheter tube 104B and engaged with the groove-shaped acceptance portion 14. Thereby, the distal end 120 of the catheter tube 104B is connected to the wireless imaging capsule 100B so as not to be easily disengaged therefrom, and a lumen 408 of the catheter tube 104B is communicatively connected with the internal lumen 404 of the wireless imaging capsule 100B. Furthermore, the high-molecular absorbent 22 is firmly attached to the inner face of the acceptance portion 14, thereby sealed is a space between the distal end 120 of the catheter tube 104B and the internal lumen 404 of the wireless imaging capsule 100B.

When the distal end 120 of the catheter tube 104B is separated from the wireless imaging capsule 100B, the proximal end of the catheter tube 104B is passed through a lumen of an overtube 130, and the overtube 130 is advanced along the catheter tube 104B. When the distal end of the overtube 130 is brought into contact with the wireless imaging capsule 100B, the overtube 130 is further advanced in such a way as to strongly pull the catheter tube 104B. Then, the high-molecular absorbent 22 is forcibly withdrawn from the acceptance portion 14. Thereby, the distal end 120 of the catheter tube 104B is separated from the wireless imaging capsule 100B.

According to the thus constituted capsule endoscope system, the catheter tube 104B can be easily connected to or disconnected from the wireless imaging capsule 100B. Furthermore, fluids such as water, air and gas can be supplied to the body of a patient via the catheter tube 104B and the wireless imaging capsule 100B, and water supplied into the body of the patient or body fluids such as saliva can be suctioned.

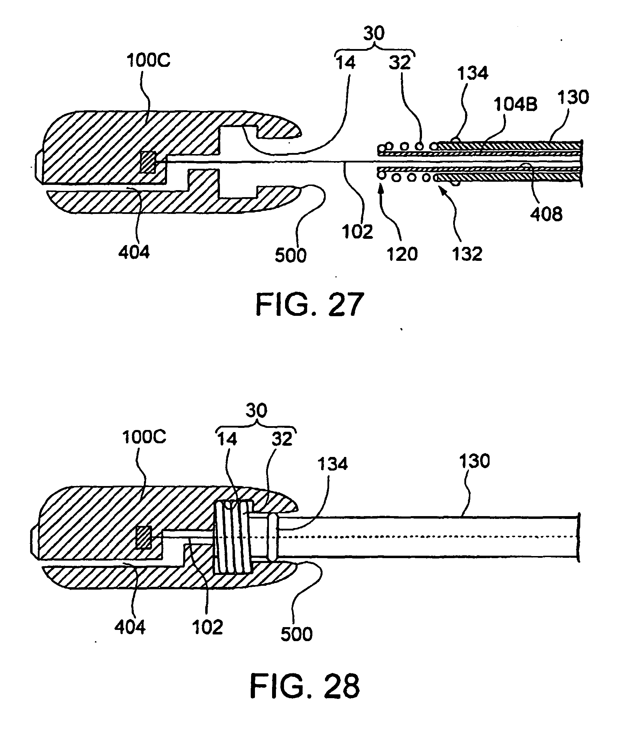

The capsule endoscope system of the present invention illustrated in FIG. 27 and FIG. 28 is provided with a capsule lumen connector (connection portion) 30. As illustrated in FIG. 27, the capsule lumen connector 30 of the present embodiment is provided with a coil (dilation member) 32 mounted on an outer peripheral face of the distal end 120 of the catheter tube 104C, an overtube 130 as a coil driving member disposed outside the catheter tube 104C so that the catheter tube 104C is passed through the overtube 130, and an acceptance portion 14.

A coil 32 is provided in such a way that one end thereof is fixed to the tip of the distal end 120 of the catheter tube 104C and the other end is fixed to the tip of the distal end 132 of the overtube 130. The coil 32 is in contact with an outer periphery of the distal end 120 of the catheter tube 104C when being free from any external actions, and undergoes an elastic deformation in such a way as to expand the outer diameter of the distal end 120 when the overtube 130 is pushed into the catheter tube 104C relatively or rotated so as to be twisted in one predetermined direction.

An annular packing 134 is fixed to an outer periphery of the distal end 132 of the overtube 130 along the circumferential direction. The packing 134 is given pressure and brought into contact with the inner circumferential face of a proximal opening 500 of the internal lumen 404 when the distal end 120 of the catheter tube 104 and the distal end 132 of the overtube 130 are inserted into the internal lumen 404 of the wireless imaging capsule 100C.

When the distal end 120 of the catheter tube 104C is connected to the wireless imaging capsule 100C, as described above, the distal end 120 of the catheter tube 104C and the distal end 132 of the overtube 130 are passed through the proximal opening 500 of the wireless imaging capsule 100C and inserted into the internal lumen 404. Then, the overtube 130 is pressed into or rotated relatively with respect to the catheter tube 104C. Thereby, as illustrated in FIG. 28, a coil 32 is subjected to an elastic deformation in such a way as to expand the outer diameter of the distal end 120 of the catheter tube 104C and thereby the coil 32 is engaged with a groove-shaped acceptance portion 14. Upon engagement of the coil 32 with an acceptance portion 14, the overtube 130 is fixed to the catheter tube 104C. Thereby, the distal end 120 of the catheter tube 104C is connected to the wireless imaging capsule 100C so as not to be easily disengaged therefrom, and a lumen 408 of the catheter tube 104C is communicatively connected with the internal lumen 404 of the wireless imaging capsule 100C. Furthermore, the packing 134 is given pressure and brought into contact with an inner peripheral face of the proximal opening 500 of the internal lumen 404, thereby sealed is a space between the distal end 132 of the overtube 130 and the internal lumen 404 of the wireless imaging capsule 100C.

When the distal end 120 of the catheter tube 104C is separated from the wireless imaging capsule 100C, the overtube 130 is released from the catheter tube 104C. Then, the coil 32 resumes its original configuration due to the intrinsic elastic force, by which the coil 32 is disengaged from the acceptance portion 14. Thereafter, only the catheter tube 104C may be pulled out. Thereby, the distal end 120 of the catheter tube 104C is separated from the wireless imaging capsule 100C.

According to the thus constituted capsule endoscope system, the catheter tube 104C can be easily connected to or disconnected from the wireless imaging capsule 100C. Furthermore, fluids such as water, air and gas can be supplied to the body of a patient via the catheter tube 104C and the wireless imaging capsule 100C, and water supplied into the body of the patient or body fluids such as saliva can be suctioned.

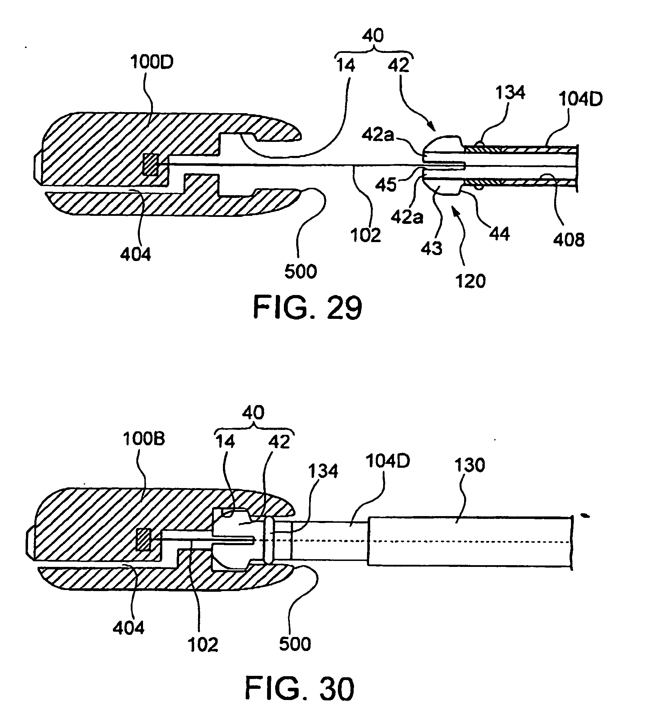

The capsule endoscope system of the present invention illustrated in FIG. 29 and FIG. 30 is provided with a capsule lumen connector (connection portion) 40. As illustrated in FIG. 29, the capsule lumen connector 40 of the present embodiment is provided with a snap portion 42 mounted on the distal end 120 of the catheter tube 104D and an acceptance portion 14.

The snap portion 42 is provided with a diameter expanding portion 43 which is formed so as to gradually expand the diameter thereof from the leading end face toward the proximal end of the catheter tube 104D. In other words, the diameter expanding portion 43 has a cross section which is formed in a wedge shape tapered toward the leading end face of the snap portion 42, and a step portion 44, the diameter of which is precipitously expanded, is formed at a part close to the proximal end of the catheter tube 104D. Furthermore, the outer diameter of the tip of the snap portion 42 is smaller than the inner diameter of the proximal opening 500 of the internal lumen 404 of the wireless imaging capsule 100D, whereas the outer diameter of the step portion 44 is larger than the inner diameter of the proximal opening 500 of the internal lumen 404.

The snap portion 42 is provided with four slits 45 formed so as to be separated at an equal space along the circumferential direction. Each of these slits 45 is notched from the leading end face of the snap portion 42 to the proximal end of the catheter tube 104D. The leading end of the snap portion 42 is provided with these slits 45, by which the snap portion 42 is divided into four parts 42a. The snap portion 42 is changed in the outer diameter due to a fact that each of the parts 42a undergoes an elastic deformation toward a radial direction of the snap portion 42.

An annular packing 134 is fixed to an outer periphery of the distal end 120 of the catheter tube 104D along the circumferential direction. Upon insertion of the distal end 120 of the catheter tube 104D into the internal lumen 404 of the wireless imaging capsule 100D, the packing 134 is given pressure and brought into contact with the inner circumferential face of the proximal opening 500 of the internal lumen 404.

When the distal end 120 of the catheter tube 104D is connected to the wireless imaging capsule 100D, the proximal end of the string tether 102 is at first passed through the lumen 408 of the catheter tube 104D, and then the catheter tube 104D is advanced along the string tether 102. When the distal end 120 of the catheter tube 104D is brought into contact with the wireless imaging capsule 100D, the catheter tube 104D is further advanced in such a way as to pull the string tether 102. Thereby, a snap portion 42 is passed through the proximal opening 500 of the wireless imaging capsule 100D and inserted into the internal lumen 404. In this instance, each of the parts 42a, which are four divisions of the snap portion 42, undergoes an elastic deformation due to a reaction force acting on a gently inclined face of the diameter expanding portion 43 from the inner peripheral face of the internal lumen 404, by which the snap portion 42 is temporarily decreased in the outer diameter. When the snap portion 42 is further inserted into the internal lumen 404, the diameter expanding portion 43 of the snap portion 42 is fitted into the acceptance portion 14 of the wireless imaging capsule 100D, by which the snap portion 42 is hooked onto the acceptance portion 14. Thereby, the distal end 120 of the catheter tube 104D is connected to the wireless imaging capsule 100D so as not to be easily disengaged therefrom, and the lumen 408 of the catheter tube 104D is communicatively connected with the internal lumen 404 of the wireless imaging capsule 100D. Furthermore, the packing 134 is given pressure and brought into contact with the inner peripheral face of the proximal opening 500 of the internal lumen 404, thereby sealed is a space between the distal end 120 of the catheter tube 104D and the internal lumen 404 of the wireless imaging capsule 100D.

Since the step portion 44 is formed at the diameter expanding portion 43, the snap portion 42 can be easily inserted into the internal lumen 404 but cannot be easily separated from the internal lumen 404. Thus, when the distal end 120 of the catheter tube 104D is separated from the wireless imaging capsule 100D, the overtube 130 is advanced along the catheter tube 104D in a similar manner as a case where the high-molecular absorbent 22 is used. When the distal end of the overtube 130 is brought into contact with the wireless imaging capsule 100D, the overtube 130 is further advanced in such a way as to strongly pull the catheter tube 104D. Then, the snap portion 42 is forcibly removed from the acceptance portion 14. Thereby, the distal end 120 of the catheter tube 104D is separated from the wireless imaging capsules 100D.

According to the thus constituted capsule endoscope system, the catheter tube 104D can be easily connected to or disconnected from the wireless imaging capsule 100D. Furthermore, fluids such as water, air and gas can be supplied to the body of a patient via the catheter tube 104D and the wireless imaging capsule 100D, and water supplied into the body of the patient or body fluids such as saliva can be suctioned.

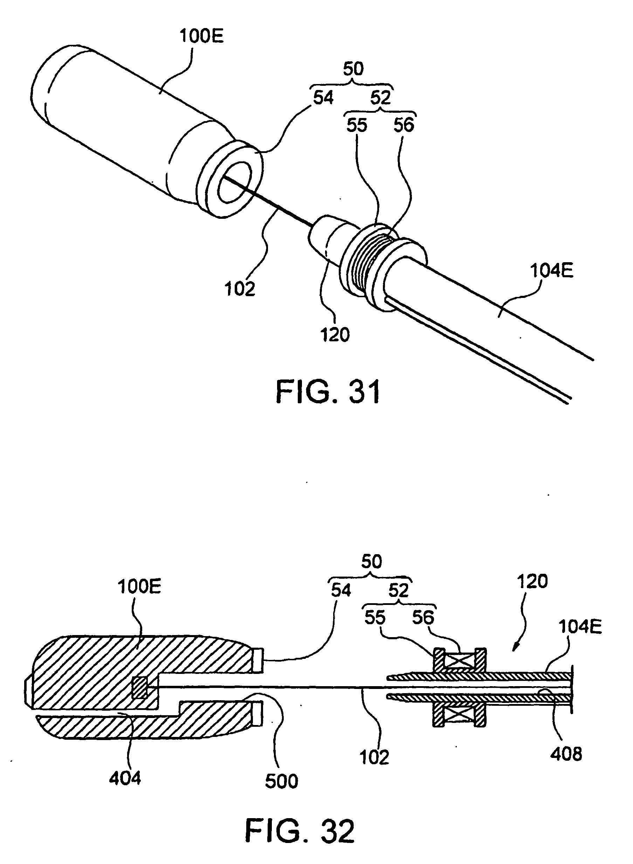

The capsule endoscope system of the present invention illustrated in FIG. 31 and FIG. 32 is provided with a capsule lumen connector (connection portion) 50. The capsule lumen connector 50 of the present embodiment is provided with an electro-magnet 52 mounted at the distal end 120 of a catheter tube 104E and a magnetic body 54 mounted on a wireless imaging capsule 100E.

The electro-magnet 52 is provided with a tubular magnet core 55 which is fixed to the distal end 120 of the catheter tube 104, with the distal end 120 being inserted inside the magnet core 55, and a conductor wire 56 coiled around the magnet core 55. When an electric current is supplied to the conductor wire 56 from a power source (not illustrated) provided separately, the electro-magnet 52 attracts the magnetic body 54 on an edge face of the magnet core 55. Then, when the electric current from the power source to the conductor wire 56 is disconnected, it releases the magnetic body 54.

The magnetic body 54 is formed in a disk shape having a hole at the center and fixed to the wireless imaging capsule 100E in such a way that the proximal opening 500 of the internal lumen 404 of the wireless imaging capsule 100E is exposed through the central hole. The edge face of the magnet core 55 facing the leading end face of the catheter tube 104E and the side face of the magnetic body 54 facing outward are both flat. When an electric current is supplied to the electro-magnet 52, the electro-magnet 52 is firmly attached to the magnetic body 54, with no clearance left.
In addition, the distal end 120 of the catheter tube 104E is tapered in such a way that the diameter thereof is made smaller as the catheter tube 104E comes closer to the leading end face.

When the distal end 120 of the catheter tube 104E is connected to the wireless imaging capsule 100E, as described above, the distal end 120 of the catheter tube 104E is passed through the proximal opening 500 of the wireless imaging capsule 100E and inserted into the internal lumen 404, and the edge face of the magnet core 55 of the electro-magnet 52 is brought into contact with an external side face of the magnetic body 54. Then, when an electric current is supplied to the conductor wire 56 of the electro-magnet 52 from the power source, the magnetic body 54 is attracted by the electro-magnet 52. Thereby, the distal end 120 of the catheter tube 104E is connected to the wireless imaging capsule 100E so as not to be easily disengaged therefrom, and the lumen 408 of the catheter tube 104 is communicatively connected with the internal lumen 404 of the wireless imaging capsule 100E. Furthermore, the edge face of the magnet core 55 of the electro-magnet 52 is firmly attached to the external side face of the magnetic body 54, with no clearance left, thereby sealed is a space between the distal end 120 of the catheter tube 104 and the internal lumen 404 of the wireless imaging capsule 100E.

When the distal end 120 of the catheter tube 104E is separated from the wireless imaging capsule 100E, an electric current supplied to the electro-magnet 52 from the power source is disconnected, and thereafter only the catheter tube 104E may be pulled out. Thereby, the distal end 120 of the catheter tube 104E is separated from the wireless imaging capsule 100E.

Incidentally, in the present embodiment, a permanent magnet may be used in place of the electro-magnet 52. In this instance, when the distal end 120 of the catheter tube 104E is connected to the wireless imaging capsule 100E, as described above, only such procedures will suffice that the distal end 120 of the catheter tube 104E is inserted into the internal lumen 404 via the proximal opening 500 of the wireless imaging capsule 100E and the permanent magnet is then brought into contact with the external side face of the magnetic body 54. When the distal end of the catheter tube 104E is separated from the wireless imaging capsule 100E, as described in a case where the high-molecular absorbent 22 is used, the overtube 130 is advanced along the catheter tube 104E, and when the distal.end of the overtube 130 is brought into contact with the wireless imaging capsule 100E, the overtube 130 is further advanced in such a way as to strongly pull the catheter tube 104E. Thereby, the permanent magnet is forcibly separated from the magnetic body 54, and the distal end 120 of the catheter tube 104E is separated from the wireless imaging capsule 100E.

According to the thus constituted capsule endoscope system, the catheter tube 104E can be easily connected to or disconnected from the wireless imaging capsule 100E. Furthermore, fluids such as water, air and gas can be supplied to the body of a patient via the catheter tube 104E and the wireless imaging capsule 100E, and water supplied into the body of the patient or body fluids such as saliva can be suctioned.

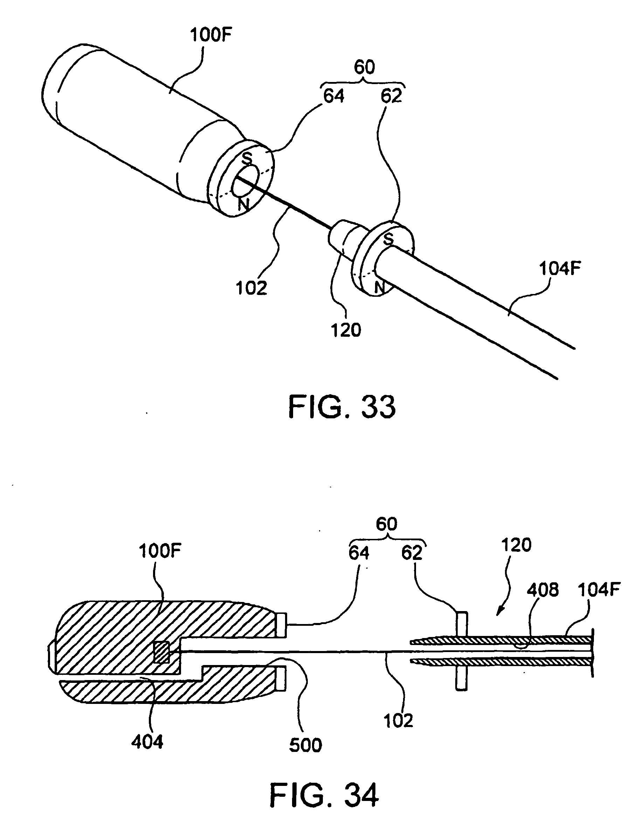

The capsule endoscope system of the present invention illustrated in FIG. 33 and FIG. 34 is provided with a capsule lumen connector (connection portion) 60. The capsule lumen connector 60 of the present embodiment is provided with a permanent magnet 62 mounted at the distal end 120 of a catheter tube 104F and a permanent magnet 64 mounted on a wireless imaging capsule 100F. It is noted that both of the magnets 62, 64 are permanent magnets.

The magnet 62 is formed in a disk shape having a hole at the center and fixed to the distal end 120 in a state where the distal end 120 of the catheter tube 104 is inserted into the central hole. Themagnet 62 is different in polarity, depending on two regions divided by the central axis of the distal end 120 of the catheter tube 104F inserted into the central hole.

The magnetic body 64 is also formed in a disk shape having a hole at the center and fixed to the wireless imaging capsule 100F in such a way that the proximal opening 500 is exposed through thecentralhole. The magnet 64 is different in polarity, depending on two regions divided by the center of the outer side face of the magnet 64, that is, the center of the proximal opening 500. The side face of the magnet 62 facing the leading end face of the catheter tube 104F and the side face of the magnetic body 64 facing outward are both flat. When the distal end 120 of the catheter tube 104F is passed through the proximal opening 500 of the wireless imaging capsule 100F and inserted into the internal lumen 404, the magnets 62, 64 are brought closer to each other and firmly attached, with no clearance left.
In addition, the distal end 120 of the catheter tube 104F is tapered so that the diameter thereof is decreased as the distal end 120 comes closer to the leading end face.

When the distal end 120 of the catheter tube 104F is connected to the wireless imaging capsule 100F, as described above, the distal end 120 of the catheter tube 104F is passed through the proximal opening 500 of the wireless imaging capsule 100F and inserted into the internal lumen 404, by which the magnets 62, 64 are attracted by each other. Thereby, the distal end 120 of the catheter tube 104F is connected to the wireless imaging capsule 100F so as not to be easily disengaged therefrom, and the lumen 408 of the catheter tube 104F is communicatively connected with the internal lumen 404 of the wireless imaging capsule 100F. Furthermore, the magnets 62, 64 are firmly attached to each other, with no clearance left, thereby sealed is a space between the distal end 120 of the catheter tube 104F and the internal lumen 404 of the wireless imaging capsule 100F. Where the N-pole of the magnet 62 is brought close to the N-pole of the magnet 64 and the S pole of the magnet 62 is brought close to the S pole of the magnet 64 upon insertion of the distal end 120 of the catheter tube 104F into the internal lumen 404 of the wireless imaging capsule 100F, there develops a repulsive force, by which either of the distal end 120 of the catheter tube 104F or the wireless imaging capsule 100F or both of them are rotated, and the N-pole of the magnet 62 and the S pole of the magnet 62 draw respectively the S pole of the magnet 64 and the N-pole of the magnet 64 and attract them.

When the distal end 120 of the catheter tube 104F is separated from the wireless imaging capsule 100F, the catheter tube 104F is rotated with respect to the string tether 102 and the magnet 62 is rotated with respect to the magnet 64. Then, the N-pole of the magnet 62 and the S pole of the magnet 62 come close respectively to the N-pole of the magnet 64 and the S pole of the magnet 64, there develops a repulsive force, by which they are separated from each other. Thereafter, only the catheter tube 104F may be pulled out. Thereby, the distal end 120 of the catheter tube 104F is separated from the wireless imaging capsule 100F.

According to the thus constituted capsule endoscope system, the catheter tube 104F can be easily connected to or disconnected from the wireless imaging capsule 100F. Furthermore, fluids such as water, air and gas can be supplied to the body of a patient via the catheter tube 104F and the wireless imaging capsule 100F, and water supplied into the body of the patient or body fluids such as saliva can be suctioned.

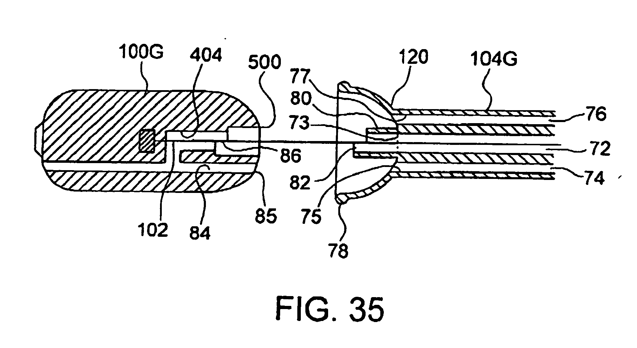

In the capsule endoscope system of the present invention illustrated in FIG. 35 to FIG. 37, a catheter tube 104G is provided with three lumens 72, 74 and 76. As illustrated in FIG. 35, of these three lumens 72, 74 and 76, the lumen 72 communicatively connected with the internal lumen 404 of the wireless imaging capsule 100G is disposed at the center of the catheter tube 104G, whereas the lumens 74, 76 are respectively disposed on both sides thereof. The distal end 120 of the catheter tube 104G is provided with a suction disc (connection portion) 78 which sucks the wireless imaging capsule 100G. The suction disc 78 is formed in a semi-spherical shape to resemble the configuration of the end portion of the wireless imaging capsule 100G at which a proximal opening 500 is formed. The distal end of the lumen 72 is opened at the center of a recessed face inside the suction disc 78 and the distal end of the lumen 74 and the distal end of the lumen 76 are opened on both sides of the distal end of the lumen 72. In other words, an opening 73 of the lumen 72 is formed at the center of the recessed face inside the suction disc 78, whereas an opening 75 of the lumen 74 and an opening 77 of the lumen 76 are formed on both sides of the opening 73.

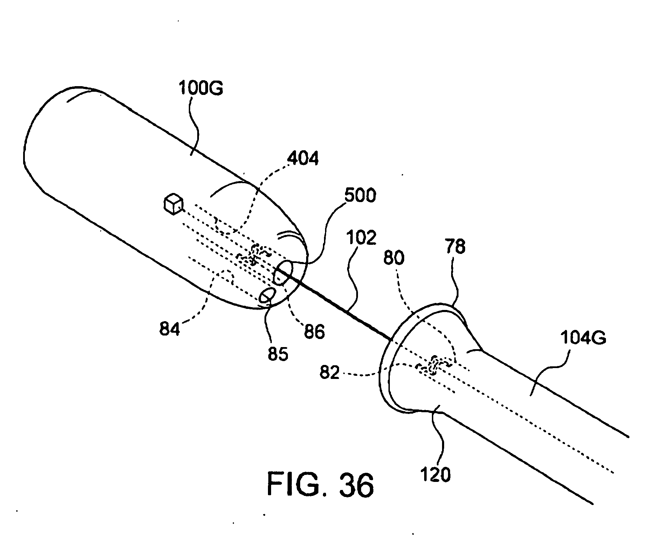

Furthermore, as illustrated in FIG. 36, a tubular portion 80 communicatively connected with the lumen 72 is formed at the center of the recessed face inside the suction disc 78 so as to project toward the longitudinal direction of the distal end 120 of the catheter tube 104G. A projection 82 which further projects toward the longitudinal direction of the tubular portion 80 is provided at the leading end of the tubular portion 80. The projection 82 is in a semi-cylindrical shape cut along the longitudinal direction of the tubular portion 80 and formed so as to leave a part of the tubular portion 80.

On the other hand, the wireless imaging capsule 100G is provided with an internal lumen 84 which is branched from the internal lumen 404 and communicatively connected with the lumen 74 when being sucked by the suction disc 78 of the catheter tube 104G, in addition to the internal lumen 404. In other words, the proximal opening 500 of the internal lumen 404 is formed at the center of the end portion which is sucked by the suction disc 78 of the wireless imaging capsule 100G, and the proximal opening 85 of the internal lumen 84 is formed on the side of the proximal opening 500.

Furthermore, the tubular portion 80 including the projection 82 is inserted into the internal lumen 404 of the wireless imaging capsule 100G via the proximal opening 500. A recess 86 which is to be engaged with the projection 82 of the tubular portion 80 is formed at the proximal opening 500. The projection 82 of the tubular portion 80 and the recess 86 of the proximal opening 500 constitute a positioning portion at which the projection 82 is used as a key to position the distal end 120 of the catheter tube 104G with respect to the wireless imaging capsule 100G.

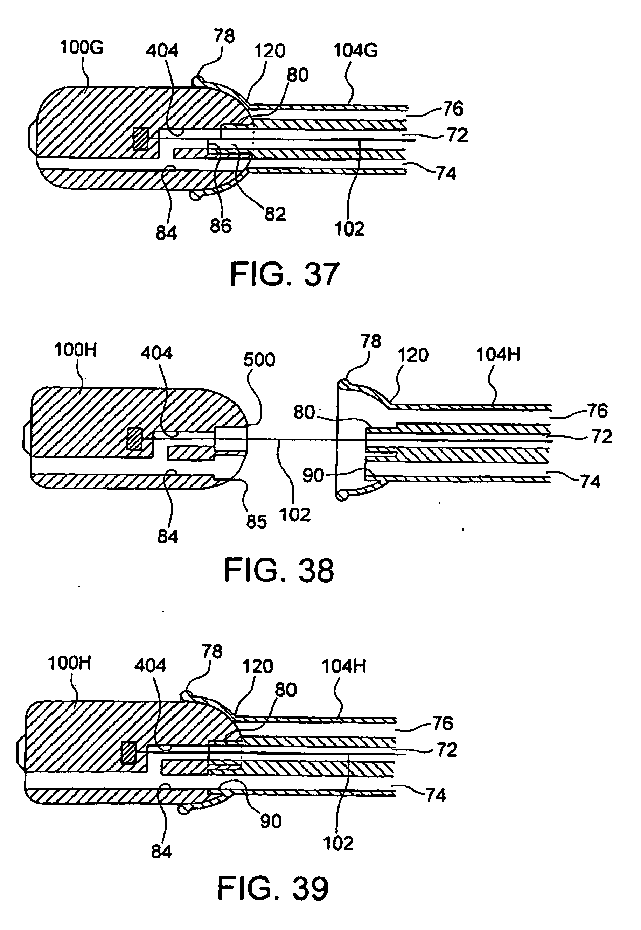

When the distal end 120. of the catheter tube 104G is connected to the wireless imaging capsule 100G, the proximal end of the string tether 102 is at first passed through the lumen 72 of the catheter tube 104G, and the catheter tube 104G is advanced along a string tether 102. Then, when the suction disc 78 of the catheter tube 104G is brought into contact with the wireless imaging capsule 100G, the catheter tube 104G is further advanced in such a way as to pull the string tether 102, and the tubular portion 80 including the projection 82 inside the suction disc 78 is passed through the proximal opening 500 of the wireless imaging capsule 100G and inserted into the internal lumen 404. In this instance, the projection 82 of the tubular portion 80 is not always engaged with the recess 86 of the proximal opening 500. Therefore, when the tubular portion 80 including the projection 82 is inserted into the internal lumen 404, the catheter tube 104G is rotated, with the catheter tube 104G being pushed. Then, even if the projection 82 has not been engaged with the recess 86, the projection 82 is soon engaged with the recess 86. As illustrated in FIG. 37, the distal end 120 of the catheter tube 104G is positioned with respect to the wireless imaging capsule 100G. Thereafter, suction is conducted via the lumen 7 6 of the catheter tube 104G, by which the wireless imaging capsule 100G is sucked to the suction disc 78. Thereby, the distal end 120 of the catheter tube 104G is connected to the wireless imaging capsule 100G so as not to be easily disengaged therefrom. The lumen 72 of the catheter tube 104C is then communicatively connected with the internal lumen 404 of the wireless imaging capsule 100G, and the lumen 74 of the catheter tube 104G is also communicatively connected with the internal lumen 84 of the wireless imaging capsule 100G. Furthermore, the end portion of the wireless imaging capsule 100G is firmly attached to the recessed face inside the suction disc 78, thereby sealed are a space between the opening 73 of the lumen 72 and the proximal opening 500 of the internal lumen 404, and also a space between the opening 75 of the lumen 74 and the proximal opening 85 of the internal lumen 84.

When the distal end 120 of the catheter tube 104G is separated from the wireless imaging capsule 100G, only the catheter tube 104G may be pulled out after suction is halted. Thereby, the distal end 120 of the catheter tube 104G is separated from the wireless imaging capsule 100G.

According to the thus constituted capsule endoscope system, the catheter tube 104G can be easily connected to or disconnected from the wireless imaging capsule 100G. Furthermore, fluids such as water, air and gas can be supplied to the body of a patient via the catheter tube 104G and the wireless imaging capsule 100G, and water supplied into the body of a patient or body fluids such as saliva can be suctioned.

Furthermore, since the positioning portion composed of the projection 82 of the tubular portion 80 and the recess 86 of the proximal opening 500 are provided, the distal end 120 of the catheter tube 104G is accurately positioned with respect to the wireless imaging capsule 100G. Also, since the catheter tube 104G is communicatively connected with the internal lumens 404 and 46 of the wireless imaging capsule 100G, procedures such as air supply, water supply and suction can be securely performed via the catheter tube 104G and the internal lumens 404 and 46 of the wireless imaging capsule 100G.

In the capsule endoscope system of the present invention illustrated in FIG. 38 and 39, a tubular portion 80 free of the projection 82 is formed at the center of the recessed face inside the suction disc 78 and also a tubular portion 90 communicatively connected with the lumen 74 is formed so as to project toward the longitudinal direction of the distal end 120 of a catheter tube 104H. As illustrated in FIG. 38, the tubular portion 90 is provided at a position eccentric to the center of the catheter tube 104H. The tubular portion 90 and the proximal opening 85 of the internal lumen 84 constitutes a positioning portion which positions the distal end 120 of the catheter tube 104H with respect to the wireless imaging capsule 100H.

When the distal end 120 of the catheter tube 104H is connected to the wireless imaging capsule 100H, as described above, the catheter tube 104H is advanced along the string tether 102. When the distal end 120 of the catheter tube 104H is brought into contact with the wireless imaging capsule 100H, the catheter tube 104H is rotated, with the catheter tube 104H being pushed. Then, the tubularportion 90 is rotated around the proximal opening 500, and at a time when the tubular portion 90 is made coincident axially with the proximal opening 85, the tubular portion 80 and the tubular portion 90 are inserted respectively into the proximal opening 500 and the proximal opening 85. Thereby, the distal end 120 of the catheter tube 104H is positioned with respect to the wireless imaging capsule 100H. Thereafter, suction is conducted through the lumen 76 of the catheter tube 104H, by which the wireless imaging capsule 100H is sucked to the suction disc 78. Thereby, the distal end 120 of the catheter tube 104H is connected to the wireless imaging capsule 100H so as not to be easily disengaged therefrom. The lumen 72 of the catheter tube 104 is communicatively connected with the internal lumen 404 of the wireless imaging capsule 100H, and the lumen 74 of the catheter tube 104H is also communicatively connected with the internal lumen 84 of the wireless imaging capsule 100H. Furthermore, an end portion of the wireless imaging capsule 100H is firmly attached to the recessed face inside the suction disc 78, thereby sealed are a space between the opening 73 of the lumen 72 and the proximal opening 500 of the internal lumen 404 and also a space between the opening 75 of the lumen 74 and the proximal opening 85 of the internal lumen 84.

When the distal end 120 of the catheter tube 104H is separated from the wireless imaging capsule 100H, only the catheter tube 104H may be pulled out after suction is halted. Thereby, the distal end 120 of the catheter tube 104H is separated from the wireless imaging capsule 100H.

According to the thus constituted capsule endoscope system, the catheter tube 104H can be easily connected to or disconnected from the wireless imaging capsule 100H. Furthermore, fluids such as water, air and gas can be supplied to the body of a patient via the catheter tube 104H and the wireless imaging capsule 100H, and water supplied into the body of the patient or body fluids such as saliva can be suctioned.

Incidentally, in the present embodiment, the tubular portion 90 may be tapered toward the leading end thereof, and the proximal opening 85 may be tapered toward the inside of the wireless imaging capsule 100H. Thereby, the tubular portion 90 can be easily inserted into the proximal opening 85. Similarly, the tubular portion 80 may be tapered and the proximal opening 500 may also be tapered.

A description has been made above for preferred embodiments of the present invention, however, the present invention shall not be limited thereto. The present invention may be subjected to addition, omission, replacement and other modifications of the constitution within a scope of the present invention. The present invention shall not be limited to the above description but will be limited only by the scope of the attached Claims.

### INDUSTRIAL APPLICABILITY

The present invention relates to a capsule endoscope system including a capsule endoscope having an internal lumen, catheter tube connected to the capsule endoscope and a connection portion for connecting the distal end of the catheter tube to the capsule endoscope so that the catheter tube is communicatively connected with the internal lumen of the capsule endoscope. According to the capsule endoscope systemof the present invention, the functionality of a swallowing-type capsule endoscope can be raised to improve the quality of an examination. A patient is also relieved of discomfort during the examination.

## Claims

1. A capsule endoscope system comprising:
a capsule endoscope (100) having an internal lumen (404);
a catheter tube (104) connected to the capsule endoscope (100);
a connection portion capable of connecting the distal end (120) of the catheter tube (104) to the capsule endoscope (100) so that the catheter tube (104) is communicatively connected with the internal lumen (404) of the capsule endoscope (100); and
a string tether (102) of which one end thereof is fixed to the capsule endoscope (100),
**characterized in that**
the catheter tube (104) is adapted to be advanced along the string tether (102), with the string tether (102) passed through the inside of the catheter tube (104) so that the string tether (102) is inserted to the inside of the catheter tube (104) with the one end being first, and thereby the catheter tube (104) is guided up to the capsule endoscope (100).

2. The capsule endoscope system according to Claim 1, wherein
the connection portion is provided with a dilation member (12; 22) mounted at the distal end of the catheter tube (104A) and capable of dilating as appropriate and an acceptance portion (14) mounted on the internal lumen (404) of the capsule endoscope (100B) and in which the acceptance portion (14) is capable of accepting the dilation portion (12; 22), and wherein
the dilation member (12; 22) is subjected to dilation within the acceptance portion (14) and thereby the dilation member is engaged with the acceptance portion (14).

3. The capsule endoscope system according to Claim 2, wherein
the dilation member (12) is a balloon capable of inflating on introduction of a fluid.

4. The capsule endoscope system according to Claim 2, wherein
the dilation member (12) is a high-molecular absorbent which dilates on supply of water.

5. The capsule endoscope system according to Claim 2, wherein
the dilation member is a coil (32) mounted at the distal end of the catheter tube and which expands the outer diameter thereof when being driven in a compressing or twisting manner, and wherein
the capsule endoscope system further comprising:
a coil driving member (130) capable of rotating the coil (32) in a compressing or twisting manner.

6. The capsule endoscope system according to Claim 5, wherein
the coil driving member (130) is an overtube disposed outside the catheter tube (104B) so that the catheter tube (104B) is passed through the overtube (130), and wherein
one end of the coil (32) is fixed to the distal end of the catheter tube (104B), while the other end of the coil (32) is fixed to the distal end of the overtube (130).

7. The capsule endoscope system according to Claim 1, wherein
the connection portion is provided with an elastically deformable snap portion (42) mounted at one of the distal end of the catheter tube (104D) and the capsule endoscope (100D), and an acceptance portion (14) mounted at the other of the distal end of the catheter tube (104D) and the capsule endoscope (100D) and hooked upon acceptance of the snap portion (42).

8. The capsule endoscope system according to Claim 1, wherein
the connection portion (50) is provided with a magnet (52; 62) mounted at one of the distal end of the catheter tube (104E; 104F) and the capsule endoscope (100E; 100F), and a magnetic body (54) mounted at the other of the distal end of the catheter tube (104E; 100F) and the capsule endoscope (100E; 100F), further wherein the magnetic body (54) is capable of being attracted by the magnet (52; 62).

9. The capsule endoscope system according to Claim 8, wherein the magnet (52) is an electro-magnet.

10. The capsule endoscope system according to Claim 8, wherein the magnet (62) is a permanent magnet.

11. The capsule endoscope system according to Claim 10, wherein
the magnetic body (64) is also a permanent magnet, the permanent magnet mounted at the distal end of the catheter tube (104F) is different in polarity depending on two regions divided by the central axis of the catheter tube (104F), and the permanent magnet (64) mounted on the capsule endoscope (100F) is different in polarity depending on two regions divided by the center of the face attached to the permanent magnet (62) mounted at the distal end of the catheter tube (104F).

12. The capsule endoscope system according to Claim 1, wherein
a positioning portion (500) capable of positioning the catheter tube (104G) with respect to the capsule endoscope (100G) is mounted at the connection portion so that to cause the catheter tube (104G) to be communicatively connected with the internal lumpen (404) of the capsule endoscope (100G).

13. The capsule endoscope system according to Claim 1, wherein
the capsule endoscope (100) is tapered toward the proximal end thereof to which the catheter tube (104) is connected.

14. The capsule endoscope system according to Claim 1, further comprising:
an operation portion (414, 416, 418) mounted at the proximal end of the catheter tube and capable of operating at least any one of the procedures of air supply, water supply and suction via the catheter tube (104) and the capsule endoscope (100) connected to the catheter tube (104).

15. The capsule endoscope system according to Claim 1, wherein
the connection portion is capable of removably connecting the distal end of the catheter tube to the capsule endoscope.

## Patentansprüche

1. Kapsel-Endoskopsystem mit:
einem Kapselendoskop (100) mit einem internen Lumen (404);
einem mit dem Kapselendoskop (100) verbundenen Katheterschlauch (104);
einem Verbindungsabschnitt, der dazu ausgebildet ist das distale Ende (120) des Katheterschlauchs (104) mit dem Kapselendoskop (100) zu verbinden, so dass der Katheterschlauch (104) mit dem internen Lumen (404) des Kapselendoskops (100) in Kommunikationsverbindung steht; und
eine Halteschnur (102), deren eines Ende an dem Kapselendoskop (100) befestigt ist,
**dadurch gekennzeichnet, dass**
der Katheterschlauch (104) entlang der Halteschnur (102) vorgeschoben zu werden vermag, wobei die Halteschnur (102) so durch das Innere des Katheterschlauchs (104) geführt ist, dass die Halteschnur (102) mit einem Ende voran in das Innere des Katheterschlauchs (104) eingeführt ist, und der Katheterschlauch (104) so bis zu dem Kapselendoskop (100) geführt wird.

2. Kapsel-Endoskopsystem nach Anspruch 1, wobei
der Verbindungsabschnitt ein an dem distalen Ende des Katheterschlauchs (104A) angebrachtes Dilatationselement (12; 22), das sich im erforderlichen Umfang weiten lässt, und einen an dem internen Lumen (404) des Kapselendoskops (100B) angebrachten Aufnahmebereich (14) aufweist, wobei der Aufnahmebereich (14) in der Lage ist, das Dilatationselement (12; 22) aufzunehmen, und wobei
das Dilatationselement (12; 22) innerhalb des Aufnahmebereichs (14) geweitet wird und **dadurch** das Dilatationselement mit dem Aufnahmebereich (14) in Eingriff gebracht wird.

3. Kapsel-Endoskopsystem nach Anspruch 2, wobei
das Dilatationselement (12) ein Ballon ist, der in der Lage ist sich aufzublähen, wenn ein Fluid in ihn eingebracht wird.

4. Kapsel-Endoskopsystem nach Anspruch 2, wobei
das Dilatationselement (12) ein hochmolekulares absorbierendes Material ist, das sich bei Zufuhr von Wasser ausdehnt.

5. Kapsel-Endoskopsystem nach Anspruch 2, wobei
das Dilatationselement eine an dem distalen Ende des Katheterschlauchs angebrachte Spirale (32) ist, deren Außendurchmesser sich vergrößert, wenn sie so angetrieben wird, dass sie zusammengedrückt oder verdreht wird, und wobei
das Kapsel-Endoskopsystem ferner umfasst:
ein Spiralenantriebselement (130), das dazu ausgebildet ist die Spirale (32) so in Drehung zu versetzen, dass sie zusammengedrückt oder verdreht wird.

6. Kapsel-Endoskopsystem nach Anspruch 5, wobei
das Spiralenantriebselement (130) ein außerhalb des Katheterschlauchs (104B) so angeordneter Übertubus ist, so dass sich der Katheterschlauch (104B) durch den Übertubus (130) erstreckt, und wobei
ein Ende der Spirale (32) an dem distalen Ende des Katheterschlauchs (104B) befestigt ist, während das andere Ende der Spirale (32) an dem distalen Ende des Übertubus (130) befestigt ist.

7. Kapsel-Endoskopsystem nach Anspruch 1, wobei
der Verbindungsabschnitt einen elastisch verformbaren Einrastbereich (42) aufweist, der entweder an dem distalen Ende des Katheterschlauchs (104D) oder an dem Kapselendoskop (100D) angebracht ist, und wobei ein Aufnahmebereich (14) an dem jeweils anderen von dem distalen Ende des Katheterschlauchs (104D) und dem Kapselendoskop (100D) angebracht ist und bei der Aufnahme des Einrastbereichs (42) einkoppelt.

8. Kapsel-Endoskopsystem nach Anspruch 1, wobei
der Verbindungsabschnitt (50) einen Magneten (52; 62) aufweist, der entweder an dem distalen Ende des Katheterschlauchs (104E; 104F) oder an dem Kapselendoskop (100E; 100F) angebracht ist, und wobei ein magnetischer Körper (54) an dem jeweils anderen von dem distalen Ende des Katheterschlauchs (104E; 100F) und dem Kapselendoskop (100E; 100F) angebracht ist, und wobei ferner der magnetische Körper (54) von dem Magneten (52; 62) angezogen zu werden vermag.

9. Kapsel-Endoskopsystem nach Anspruch 8, wobei der Magnet (52) ein Elektromagnet ist.

10. Kapsel-Endoskopsystem nach Anspruch 8, wobei der Magnet (62) ein Permanentmagnet ist.

11. Kapsel-Endoskopsystem nach Anspruch 10, wobei
der magnetische Körper (64) ebenfalls ein Permanentmagnet ist, und der an dem distalen Ende des Katheterschlauchs (104F) angebrachte Permanentmagnet abhängig von zwei durch die Mittelachse des Katheterschlauchs (104F) voneinander getrennten Bereichen eine unterschiedliche Polarität aufweist, und wobei der an dem Kapselendoskop (100F) angebrachte Permanentmagnet (64) eine unterschiedliche Polarität aufweist, abhängig von zwei Bereichen, die durch die Mitte der Fläche, die an dem an dem distalen Ende des Katheterschlauchs (104F) angebrachten Permanentmagneten (62) angefügt ist, voneinander getrennt sind.

12. Kapsel-Endoskopsystem nach Anspruch 1, wobei
ein Positionierbereich (500), der dazu ausgebildet ist, den Katheterschlauch (104G) in Bezug auf das Kapselendoskop (100G) zu positionieren, so an dem Verbindungsabschnitt angebracht ist, dass der Katheterschlauch (104G) zum Erstellen einer Kommunikationsverbindung zwischen ihm und dem internen Lumen (404) des Kapselendoskops (100G) beaufschlagt wird.

13. Kapsel-Endoskopsystem nach Anspruch 1, wobei
das Kapselendoskop (100) sich in Richtung seines proximalen Endes, mit dem der Katheterschlauch (104) verbunden ist, verjüngt.

14. Kapsel-Endoskopsystem nach Anspruch 1, ferner aufweisend:
einen an dem proximalen Ende des Katheterschlauchs angebrachten Betätigungsbereich (414, 416, 418), der dazu ausgebildet ist, zumindest einen der folgenden Vorgänge zu bewirken: Luftzufuhr, Wasserzufuhr und Saugwirkung über den Katheterschlauch (104) und das mit dem Katheterschlauch (104) verbundene Kapselendoskop (100).

15. Kapsel-Endoskopsystem nach Anspruch 1, wobei
der Verbindungsabschnitt dazu ausgebildet ist, das distale Ende des Katheterschlauchs lösbar mit dem Kapselendoskop zu verbinden.

## Revendications

1. Système d'endoscope à capsule comprenant :
un endoscope à capsule (100) comportant une lumière interne (404) ;
un tube de cathéter (104) raccordé à l'endoscope à capsule (100) ;
une partie de raccordement capable de raccorder l'extrémité distale (120) du tube de cathéter (104) à l'endoscope à capsule (100) de sorte que le tube de cathéter (104) est raccordé en communication avec la lumière interne (404) de l'endoscope à capsule (100) ; et
un fil d'attache (102) dont une extrémité de celui-ci est fixée à l'endoscope à capsule (100),
**caractérisé en ce que**
le tube de cathéter (104) est adapté pour être avancé le long du fil d'attache (102), le fil d'attache (102) passant à travers l'intérieur du tube de cathéter (104) de sorte que le fil d'attache (102) est inséré vers l'intérieur du tube de cathéter (104) avec l'extrémité en premier, et de ce fait le tube de cathéter (104) est guidé jusqu'à l'endoscope à capsule (100).

2. Système d'endoscope à capsule selon la revendication 1, dans lequel
la partie de raccordement est munie d'un élément de dilatation (12 ; 22) monté au niveau de l'extrémité distale du tube de cathéter (104A) et capable de se dilater de manière appropriée et d'une partie de réception (14) montée sur la lumière interne (404) de l'endoscope à capsule (100B) et dans laquelle la partie de réception (14) est capable de recevoir la partie de dilatation (12 ; 22), et dans lequel
l'élément de dilatation (12 ; 22) est soumis à la dilatation à l'intérieur de la partie de réception (14) et de ce fait l'élément de dilatation est engagé avec la partie de réception (14).

3. Système d'endoscope à capsule selon la revendication 2, dans lequel
l'élément de dilatation (12) est un ballon capable de gonfler sur introduction d'un liquide.

4. Système d'endoscope à capsule selon la revendication 2, dans lequel
l'élément de dilatation (12) est un absorbant à poids moléculaire élevé qui se dilate sur alimentation en eau.

5. Système d'endoscope à capsule selon la revendication 2, dans lequel
l'élément de dilatation est une bobine (32) montée au niveau de l'extrémité distale du tube de cathéter et qui augmente le diamètre externe de celui-ci lorsqu'il est entraîné dans un mode de compression ou de torsion, et dans lequel
le système d'endoscope à capsule comprenant en outre :
un élément d'entraînement de bobine (130) capable de faire tourner la bobine (32) dans un mode de compression ou de torsion.

6. Système d'endoscope à capsule selon la revendication 5, dans lequel
l'élément d'entraînement de bobine (130) est un surtube disposé à l'extérieur du tube de cathéter (104B) de sorte que le tube de cathéter (104B) est passé à travers le surtube (130), et dans lequel
une extrémité de la bobine (32) est fixée à l'extrémité distale du tube de cathéter (104B), alors que l'autre extrémité de la bobine (32) est fixée à l'extrémité distale du surtube (130).

7. Système d'endoscope à capsule selon la revendication 1, dans lequel
la partie de raccordement est munie d'une partie à encliquetage élastiquement déformable (42) montée au niveau d'une extrémité distale du tube de cathéter (104D) et de l'endoscope à capsule (100D), et d'une partie de réception (14) montée au niveau de l'autre extrémité distale du tube de cathéter (104D) et de l'endoscope à capsule (100D) et crochetée sur réception de la partie à encliquetage (42).

8. Système d'endoscope à capsule selon la revendication 1, dans lequel
la partie de raccordement (50) est munie d'un aimant (52 ; 62) monté au niveau d'une extrémité distale du tube de cathéter (104E ; 104F) et de l'endoscope à capsule (100E ; 100F), et d'un corps magnétique (54) monté au niveau de l'autre extrémité distale du tube de cathéter (104E ; 104F) et de l'endoscope à capsule (100E ; 100F), de plus, dans laquelle le corps magnétique (54) est capable d'être attiré par l'aimant (52 ; 62).

9. Système d'endoscope à capsule selon la revendication 8, dans lequel l'aimant (52) est un électroaimant.

10. Système d'endoscope à capsule selon la revendication 8, dans lequel l'aimant (62) est un aimant permanent.

11. Système d'endoscope à capsule selon la revendication 10, dans lequel le corps magnétique (64) est également un aimant permanent, l'aimant permanent monté au niveau de l'extrémité distale du tube de cathéter (104F) est de polarité différente en fonction de deux régions divisées par l'axe central du tube de cathéter (104F), l'aimant permanent (64) monté sur l'endoscope à capsule (100F) est de polarité différente en fonction de deux régions divisées par le centre de la face fixée à l'aimant permanent (62) monté au niveau de l'extrémité distale du tube de cathéter (104F).

12. Système d'endoscope à capsule selon la revendication 1, dans lequel
une partie de positionnement (500) capable de positionner le tube de cathéter (104G) par rapport à l'endoscope à capsule (100G) est montée au niveau de la partie de raccordement de façon à amener le tube de cathéter (104G) à être raccordé en communication avec la lumière interne (404) de l'endoscope à capsule (100G).

13. Système d'endoscope à capsule selon la revendication 1, dans lequel
l'endoscope à capsule (100) est conique vers l'extrémité proximale de celui-ci à laquelle le tube de cathéter (104) est raccordé.

14. Système d'endoscope à capsule selon la revendication 1, comprenant en outre :
une partie d'actionnement (414, 416, 418) montée au niveau de l'extrémité proximale du tube de cathéter et capable de réaliser au moins n'importe laquelle parmi les procédures d'alimentation en air, d'alimentation en eau et d'aspirations via le tube de cathéter (104) et l'endoscope à capsule (100) raccordé au tube de cathéter (104).

15. Système d'endoscope à capsule selon la revendication 1, dans lequel
la partie de raccordement est capable de raccorder de manière amovible l'extrémité distale du tube de cathéter à l'endoscope à capsule.
